# EUROPEAN PATENT SPECIFICATION

(11) **EP 0 572 560 B1**
(45) Date of publication and mention of the grant of the patent: **29.01.2003**
(21) Application number: 92907973.9
(22) Date of filing: 21.02.1992
(51) Int. Cl.: A61K 31/34, A61K 31/505, A61K 31/54, A61K 31/165, A61K 31/205, A61K 31/14, A61K 31/715, A61K 31/18, A61K 31/195, A61P 25/00, A61P 21/00

(54) **USE OF PHARMACEUTICAL COMPOUNDS IN THE TREATMENT OF SYMPTOMS OF DISORDERS RELATED TO NEUROLOGICAL DISEASES AND ETIOLOGICALLY RELATED SYMPTOMOLOGY**
VERWENDUNG VON PHARMAZEUTISCHEN WIRKSTOFFEN ZUR BEHANDLUNG VON KRANKHEITSSYMPTOMEN NEUROLOGISCHER ERKRANKUNGEN UND ETIOLOGISCH VERWANDTER SYMPTOMENKOMPLEXE
UTILISATION DE COMPOSES PHARMACEUTIQUES POUR LE TRAITEMENT DE TROUBLES ASSOCIES A DES AFFECTIONS NEUROLOGIQUES ET A DES SYMPTOMES APPARENTES DE FA ON ETIOLOGIQUE

(30) Priority: 22.02.1991 US 660561
(43) Date of publication of application: 08.12.1993
(73) Proprietor: SHAPIRO, Howard, K., Narberth, PA 19072 (US)
(72) Inventor: SHAPIRO, Howard, K., Narberth, PA 19072 (US)
(74) Representative: O'Connor, Donal Henry
(86) International application number: US9201365
(87) International publication number: WO92014456

(56) References cited:
- WO-A-89/03216
- US-A- 3 895 107
- US-A- 4 039 388
- US-A- 4 154 850
- US-A- 4 309 534
- US-A- 4 595 586
- US-A- 4 638 014
- US-A- 4 665 069
- US-A- 4 812 447
- US-A- 4 889 722
- US-A- 4 923 696
- US-A- 5 013 769
- WINDHOLZ ET AL. 'The Merck Index' , MERCK & CO. , RAHWAY, US * page 860, abstract no. 5344 *
- WINDHOLZ ET AL. 'The Merck Index' , MERCK & CO. , RAHWAY, US * page 60, abstract no. 380 *
- TORTORA & ANGNOSTAKOS, "Principles of Anatomy & Physiology", 2nd Edition, published 1975 by Harper & Row Publishers Inc., see pages 394 and 589.
- ZUBAY, "Biochemistry", published 1983 by the Benjamin/Cummings Publishing Company, Inc., see pages 565,1104-1109 and 1115.

## Description

### I. SUMMARY OF THE INVENTION

The present invention is directed to the use of a primary agent as described below which is a water soluble, low molecular weight substance containing a primary amine group, for preparing a pharmaceutical composition intended for oral administration for treating the symptoms of disorders based on neurofilament associated pathology and/or pathophysiologically related symptomology.

In a preferred embodiment, the use of a primary agent which is a water soluble, low molecular weight substance in the range of 100 to 1,100 is selected from the group consisting of free acid forms, pharmaceutically acceptable salts, benzene ring isomers, carboxylic acid ester derivatives and sulfonic acid ester derivatives of the group consisting of:
a. p-aminobenzoic acid (PABA);
b. p-aminomethylbenzoic acid and analogous derivatives of the formula **H**_{**2**} **N-(CH**_{**2**}**)**_{**n**} **-C**_{**6**} **H**_{**4**} **-COOH** where n = 2-30, including m- and o-benzene ring isomers of the aminoalkyl group and isomers of the aminoalkyl group where the amine is not in the ω position;
c. 4-Amino-3-methylbenzoic acid and other derivatives of PABA or benzene ring isomers thereof wherein such derivatives include from one to four additional ring substituents from the group consisting of methyl group(s), ethyl group(s), or other hydro-carbongroup(s) (up to 5 carbons); or substituted -OH group(s) of the structure -OCH₃, -C₂H₅ or higher molecular weight ethers (up to 5 carbons);
d. 4-amidinobenzoic acid, **H**_{**2**} **N-C(=NH)C**_{**6**} **H**_{**4**} **-COOH,** and the following derivatives thereof: 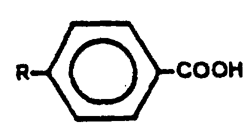 where **R= -NHC(=NH)NH**_{**2**}**, CH**_{**2**} **NHC(=NH)NH**_{**2**}
   or **(CH**_{**2**} **)**_{**n**} **NHC(-NH)NH**_{**2**} where n = 2 - 10;
e. p-aminophenylacetic acid and analogous derivatives of the formula **H**_{**2**} **N-(CH**_{**2**}**)**_{**n**} **-C**_{**6**} **H**_{**4**} **-CH**_{**2**} **-COOH** where n = 1-30, as well as methyl and other sidechain hydrocarbon isomers of the aminoalkyl group, and/or hydroxylated derivatives of the sidechain amino-alkyl group, and/or derivatives bearing hydrocarbon or hydroxy substitutions at the α carbon of the acetate group;
f. 4-amidinophenylacetic acid, **H**_{**2**} **N-C(=NH)C**_{**6**} **H**_{**4**} **-CH**_{**2**} **-COOH;**
g. 3,5-diaminobenzoic acid and other benzene ring diamine isomers;
h. 3,5-diaminoalkylbenzoic acid and benzene ring isomers, where aminoalkyl is **H**_{**2**} **N-(CH**_{**2**}**)**_{**n**}**-** and n = 1-30, including hydro-carbon isomers, or where aminoalkyl is **H**_{**2**} **N-(CH**_{**2**}**)**_{**m**}**-CHOH-(CH**_{**2**}**)**_{**n**}**-** where m = 0 - 15 and n = 0 - 15, including hydrocarbon isomers thereof;
i. p-aminosalicylic acid, and the isomeric amine and hydroxy derivatives thereof, as well as derivatives wherein the hydroxy group has been replaced by a methoxy group or alkyloxy group having 2-10 carbons;
j. 4-amino-2-sulfobenzoic acid, and derivatives thereof including benzene ring isomers and derivatives where the amino group is replaced by an aminoalkyl group having 1-10 carbons, and derivatives where the carboxylic acid group is replaced by a **-(CH**_{**2**} **)**_{**n**} **-COOH** group (n=1-10);
   for preparing a pharmaceutical composition intended for oral administration for treating the symptoms of hereditary motor and sensory neuropathies; giant axonal neuropathy; diabetic polyneuropathy or metabolic symptomology related thereto; Alzheimer's presenile dementia; Alzheimer's senile dementia; Down's syndrome; Pick's disease; Parkinson's disease; amyotrophic lateral sclerosis; disorders clinically related to the aforementioned neurological diseases; Huntington's disease; tinnitus; spinal muscular atrophy; Friedreich's ataxia; alcoholic polyneuropathy; multiple sclerosis; ceroid lipofuscinosis; age-related atrophy of peripheral sensory and motor nerves; age-related atrophy of autonomic nerves including symptoms of hypoperistalsis of the alimentary tract, hiatal hernia (partial food regurgitation), urinary incontinence, breathing insufficiency due to diaphragm weakness and decreased autonomic sexual function; age-related atrophy of neurons of the central nervous system; muscular dystrophy disorders; age-onset pathophysiologically related changes in the cardiovascular system, kidney and optic lens; or atherosclerosis.

In a preferred embodiment the primary agent which is a water soluble low molecular weight substance is used in a dosage in the range of 600 mg/day to 40 grams/day.

In a required embodiment the use of the primary agent which is a water soluble low molecular weight substance is used in combination with a co-agent selected from the closed group of antioxidants consisting of vitamin E (α-tocopherol), selenium, citric acid, ubiquinol, a seleno-containing amino acid, glutathione and sulfhydryl containing proteins, the closed group of vitamins consisting of vitamin A, vitamin D, vitamin K and vitamin B-6, the closed group of hormone consisting of human growth hormone, the closed group of chemical conjugating substance which facilitates kidney drug elimination consisting of glycine, the closed group of metabolite at risk of depletion consisting of pantothenic acid, the closed group of sulfhydryl containing substances consisting of cysteine, homocysteine, methionine and thioctic acid (α-lipoic acid).

In another aspect of the invention, the invention relates to the use of a primary agent together with a co-agent as above to effectively compete with and covalently bind to disease-induced carbonyl-containing aliphatic or aromatic hydrocarbons for use in the treatment of symptoms of disorders based on neurological disease characterized by the deterioration of intracellular structures and by the spurious pathological chemical crosslinking of intracellular structures, wherein the deterioration and the crosslinking results from reaction of nerve cells and intracellular structures with dis-ease-induced carbonyl-containing aliphatic or aromatic hydro-carbons, wherein the chemical crosslinking comprises covalent bond crosslinking of the intracellular structures.

In such a preferred embodiment of the use of the present invention, the covalent bond crosslinking of the intracellular structure additionally comprises a neuropathological structure(s) selected from the group consisting of:
a. polymerized aggregates of structural protein filaments such as excess neurofilament accumulation;
b. heterogeneous protein aggregates such as neurofibrillary tangles;
c. amorphous protein and lipid aggregates, such as senile plaques; or
d. lipofuscin granules.

In a preferred embodiment of this aspect of the invention, the use of the primary agent characterized as a water soluble, small molecular weight chemical having at least one primary amine group thereon is for reaction with carbonyl groups to yield covalently bonded products, and wherein the low molecular weight primary agent is selected from the group as defined above.

In a preferred embodiment, the use of such a primary agent is characterized in that the agent does not interact with the normal cell metabolism or does so in a non-cytotoxic manner, is capable of being tolerated in dosages in the range of 600 mg/day to 40 grams/day for extended periods of time and wherein the agent is readily absorbed by the kidney tissue and excreted in the urine without nephrotoxic consequences. In a required embodiment, the use additionally comprises the use of a co-agent selected from closed groups consisting of antioxidants, a hormone, suspending reagents, vitamins, a chemical conjugating substance, a metabolite at risk of depletion and sulfhydryl containing substances.

In another aspect of the invention, the invention relates to a pharmaceutical composition intended for oral administration for treating the symptoms of: hereditary motor and sensory neuropathies; giant axonal neuropathy; diabetic polyneuropathy or metabolic symptomology related thereto; Alzheimer's presenile dementia; Alzheimer's senile dementia; Down's syndrome; Pick's disease; Parkinson's disease; amyotrophic lateral sclerosis; disorders clinically related to the aforementioned neurological diseases; Huntington's disease; tinnitus; spinal muscular atrophy; Friedreich's ataxia; alcoholic polyneuropathy; multiple sclerosis; ceroid lipofuscinosis; age-related atrophy of peripheral sensory and motor nerves; age-related atrophy of autonomic nerves including symptoms of hypoperistalsis of the alimentary tract, hiatal hernia (partial food regurgitation), urinary incontinence, breathing insufficiency due to diaphragm weakness and decreased autonomic sexual function; age-related atrophy of neurons of the central nervous system; muscular dystrophy disorders; age-onset pathophysiologically related changes in the cardiovascular system, kidney and optic lens; or atherosclerosis, the composition comprising one or more primary agents selected from: free acid forms, pharmaceutically acceptable salts, benzene ring isomers, carboxylic acid ester derivatives and sulfonic acid ester derivatives of the group consisting of:
a. p-aminobenzoic acid (PABA);
b. p-aminomethylbenzoic acid and analogous derivatives of the formula **H**_{**2**} **N-(CH**_{**2**}**)**_{**n**} **-C**_{**6**} **H**_{**4**} **-COOH** where n = 2-30, including m- and o-benzene ring isomers of the aminoalkyl group and isomers of the aminoalkyl group where the amine is not in the ω position;
c. 4-Amino-3-methylbenzoic acid and other derivatives of PABA or benzene ring isomers thereof wherein such derivatives include from one to four additional ring substituents from the group consisting of methyl group(s), ethyl group(s), or other hydro-carbon group(s) (up to 5 carbons); or substituted -OH group(s) of the structure -OCH₃, -C₂H₅ or higher molecular weight ethers (up to 5 carbons);
d. 4-amidinobenzoic acid, **H**_{**2**} **N-C(=NH)C**_{**6**} **H**_{**4**} **-COOH,** and the following derivatives thereof: 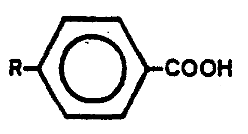 where
   **R= -NHC(=NH)NH**_{**2**}**, CH**_{**2**} **NHC(=NH)NH**_{**2**} **or (CH**_{**2**} **)**_{**n**} **NHC(=NH)NH**_{**2**}
   where n = 2 - 10;
e. p-aminophenylacetic acid and analogous derivatives of the formula **H**_{**2**} **N-(CH**_{**2**}**)**_{**n**} **-C**_{**6**} **H**_{**4**} **-CH**_{**2**} **-COOH** where n = 1-30, as well as methyl and other sidechain hydrocarbon isomers of the aminoalkyl group, and/or hydroxylated derivatives of the sidechain aminoalkyl group, and/or derivatives bearing hydrocarbon or hydroxy substitutions at the α carbon of the acetate group;
f. 4-amidinophenylacetic acid, **H**_{**2**} **N-C(=NH)C**_{**6**} **H**_{**4**} **-CH**_{**2**} **-COOH;**
g. g. 3,5-diaminobenzoic acid and other benzene ring diamine isomers;
h. 3,5-diaminoalkylbenzoic acid and benzene ring isomers, where aminoalkyl is **H**_{**2**} **N-(CH**_{**2**}**)**_{**n**}**-** and n = 1-30, including hydro-carbon isomers, or where aminoalkyl is **H**_{**2**} **N-(CH**_{**2**}**)**_{**m**}**-CHOH-(CH**_{**2**}**)**_{**n**}**-** where m = 0 - 15 and n = 0 - 15, including hydrocarbon isomers thereof;
i. p-aminosalicylic acid, and the isomeric amine and hydroxy derivatives thereof, as well as derivatives wherein the hydroxy group has been replaced by a methoxy group or alkyloxy group having 2-10 carbons;
j. 4-amino-2-sulfobenzoic acid, and derivaties thereof including benzene ring isomers and derivatives where the amino group is replaced by an aminoalkyl group having 1-10 carbons, and derivatives where the carboxylic acid group is replaced by a **-(CH**_{**2**}**)**_{**n**} **-COOH** group (n=1-10); in a dosage rate of from 600 milligrams/day to 40 grams/day, in association with a pharmaceutically acceptable carrier thereof.

In a required embodiment, this pharmaceutical composition additionally comprises the use of a co-agent selected from closed groups consisting of antioxidants, suspending reagents or the functional equivalents thereto, vitamins, a hormone, a chemical conjugating substance, a metabolite at risk of depletion or sulfhydryl containing substances as described previously.

### II. BACKGROUND OF THE ART: DISEASE-SPECIFIC EVIDENCE OF NEUROFILAMENT ASSOCIATED ETIOLOGY

International Publication No. WO89/03216(D1) discloses the use of 5-aminosalicylic acid (5-ASA) for the production of a pharmaceutical preparation for use in reperfusion treatment in connection with coronary circulation diseases and their complications.

The Merck Index, VIII Edition, Abstract No. 5344 (D2) discloses Levodopa. The Merck Index VIII Edition, Abstract No. 380 (D3) discloses Amantadine.

U.S. Patent No. 3,895,107 (D4) discloses a method of treatment for inhibiting the development of atherosclerotic lesions in animals of the mammalian species, promoting development of collateral circulation in regions of the heart supplied by the branches of the coronary arteries, and inhibiting the occurrence of cardiac episodes including myocardial infarctions, acute coronary insufficiency with acute myocardial ischemia in human subjects with ischemic heart disease, which comprises the essentially regular and prolonged oral administration to animals of an effective amount of the biologically and physiologically "active" chondroitin sulfate A, "active" chondroitin sulfate or mixtures thereof.

U.S. Patent No. 4,638,014 (D5) discloses a method for treating or preventing convulsions in mammals comprising administration of certain aminobenzanilide compounds.

U.S. Patent No. 4, 812, 447 (D6) discloses a method for facilitating the growth of brain cells, a method for promoting the growth of the nervous system tissue, and a pharmaceutical composition for treating nervous system degeneration in a mammal caused by age or senile dementia of the Alzheimer's type comprising administration of dehydroepiandrosterone or dehydroepiandrosterone sulfate.

A method of preparing a neurotrophic protein composition and a substantially pure neurotrophic factor is disclosed in U.S. Patent No. 4,923,696 (D7).

The Merck Index, X Edition, References 423, 445, 485, 487, 2941 (D8) disclose ρ-Aminobenzoic Acid (PABA), ρ-Aminohippuric Acid (PAH), ρ-Aminosalicylic Acid (PAS), 4-Amino-2-sulfobenzoic Acid, 3, 5-Diaminobenzoic Acid.

U.S. Patent No. 4,665,069 (D9) discloses an analgesic composition, an analgesia composition in kit form and a method of analgesia all comprising an aqueous solution of a parasympathomimetic agent adapted for topical application to the genitalia.

The prior art sections herein cover information on many diseases which feature neurological damage. This includes both heredi-tary as well as acquired diseases, as well as neurological damage related to aging.

### II (A). Hereditary Motor and Sensory Neuropathies (HMSN, or Charcot-Marie-Tooth Disease)

HMSN disorders are a group of peripheral neuropathy syndromes which have been classified into at least seven types (Dyck, PJ, 1984). At least three autosomal dominant forms of HMSN have been defined; chromosome one-linked, chromosome 17-linked, and non-1/non-17.

In the HMSN II family examined by Goebel and coworkers (1986) peripheral nerve myelinated and, to a lesser extent, unmyelinated axons showed clear evidence of excess neurofilament accumulation, including spheroid (i.e., ballooned) axon segments. Some axons which contained excess neurofilaments did not show any apparent increase in diameter, prompting the investigators to suggest that earlier light microscopy studies may have overlooked this finding. Excess accumulation of neurofilaments, which forces other organelles to the edges of the cytoplasm and creates "balloon" bottlenecks along the axon, may be expected to impede normal axonal transport.

In 1973 Brimijoin and coworkers examined the rate of transport of endogenous dopamine-β-hydroxylase (DBH) in vitro in samples of normal sural nerve biopsies as well as nerve biopsies from one HMSN I patient, one HMSN II patient and one HMSN III patient. They found that the HMSN proximo-distal slow component transport rates were substantially reduced: 10% of control for HMSN I, 16% for HMSN II and no measurable rate for HMSN III. The investigators concluded that a defect is apparent in the axonal transport system of HMSN nerves. These findings are in. accord with the published hypothesis that a spectrum of neuro-logical diseases may share a common basis in being disorders of axonal transport (Tomlinson, DR and Mayer, JH, 1984).

More recently this inventor has published the findings of a study which may provide a molecular basis for the HMSN data noted above (Shapiro, HK et al., 1986; Shapiro, HK and Kahn, GC, 1990). In this study urine samples from five autosomal dominant HMSN I patients (chromosome 17 variety) of the same family and five urine samples from age- and sex-matched normal control sub-jects were examined. By use of gas chromatography/mass spec-trometry the urine concentrations of approximately 150 organic acids could be estimated in each sample. Average HMSN I organic acid values differed most notably from normal values for a set of three physiologically related metabolites, 5-hydroxymethyl-2-furoic acid, 2,5-furandi-carboxylic acid and 5-carboxy-2-furoyl-glycine. Average patient urine concentrations of these three organic acids were 29%, 50% and 37% of controls, respectively.

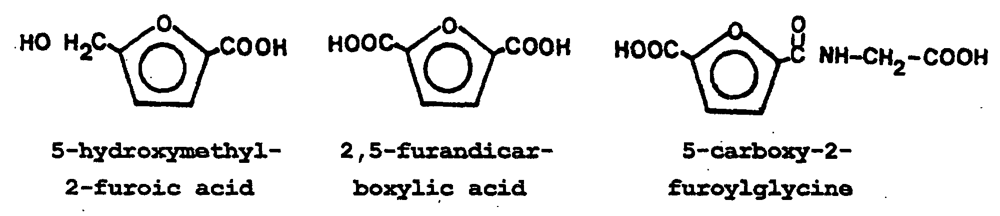

Previous research studies have determined that 5-hydroxymethyl-2-furoic acid and 2,5-furandicarboxylic acid are oxidation products of an aldehyde precursor, 5-hydroxymethyl-2-furfural (Jellum, E et al., 1973). Decreased levels of furancarboxylic acid excretion suggest that this metabolite, and possibly other aldehyde precursors such as 2,5-furandialdehyde, is not being detoxified and cleared in a normal manner.

5-Hydroxymethyl-2-furfural should be regarded as a potential protein crosslinking agent (Jellum, E et al., 1973, pg. 200). 2,5-Furandialdehyde is even more suspect as a potential crosslinking agent, as it bears two highly reactive aldehyde groups. It is a close structural analogue of 2,5-hexanedione, a potent chemical peripheral neurotoxin implicated in the covalent crosslinking of neurofilaments (structure below). Covalent chemical crosslinking of neurofilaments has been shown to be the basis of 2,5-hexanedione neurotoxicity (Carden, MJ et al., 1986)..

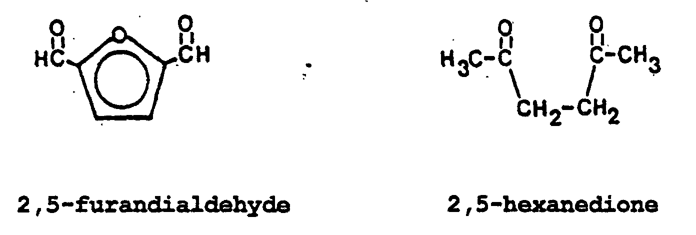

The results of a heretofore unpublished study conducted by this inventor provide additional evidence that chemical crosslinking of neurofilaments may underlie the etiology of at least some HMSN disorders. In this study the proteins of three HMSN tpye I (chromosome 17 variety) and three control skin fibroblast strains were analyzed by two dimensional gel electrophoresis and subsequent computer image analysis. The HMSN patient skin biopsies came from donors who had previously participated in the organic acid metabolic profiling study noted above. Protein mapping work was carried out at Protein Databases, Inc. (Hunt-ington Station, NY), with financial support provided by the National Foundation for Jewish Genetic Diseases (New York).

In this study 145 protein spots were always seen in each of the three normal fibroblast strains, and 126 corresponding protein spots were always seen in each of the HMSN strains. However, each of the HMSN samples also showed 24 additional protein spots which were never seen in any of the control samples. There were no examples of a protein always seen in each of the control sam-ples but never seen in any of the HMSN samples. The distribu-tion of molecular weights of the additional HMSN-specific pro-tein spots did not correspond to the molecular weight distribu-tion of control protein spots. Rather, it was comparatively shifted up scale. Of the protein spots always seen in control samples, the largest had a molecular weight of 118,000. Of the 24 HMSN-specific protein spots nine had molecular weights in the range of 130,000 to 192,000. The information available from this study can most directly be interpreted as evidence of ex-cess, pathological chemical crosslinking of fibroblast proteins.

There is reason to believe that 5-hydroxymethyl-2-furfural and 2,5-furandialdehyde can originate as by-products of either of two general areas of metabolism, that of sugars and lipids. As discussed in Section VI(B) of this text, these two furanalde-hydes form spontaneously from glucose or fructose under mildly acidic aqueous conditions and, as they are readily generated during food cooking, they are part of the human diet.

There is reason to believe that these aldehydes, among others, may play a significant role in the etiology of diabetic polyneuropathy (see Section II[C] of this text). As discussed in Section VI(C) of this disclosure, furanaldehydes may also be generated as secondary products of lipid peroxidation.

### II(B). Giant Axon Neuropathy

In the case study of Prineas and coworkers (1976) evidence of a generalized abnormality of cyto-plasmic microfilament metabolism was found. Peripheral nerves were not generally enlarged, but electron microscopy revealed numerous axonal swellings, or balloon-like structures, randomly positioned along many nerve axons. These axonal spheroids were filled with tightly packed neurofilaments. Both myelinated and unmyelinated fibers were affected.

Prineas and coworkers also noted the presence of discrete masses of cytoplasmic filaments in several other types of cells, including Schwann cells, endoneurial fibroblasts, perineurial cells, endothelial cells of endoneurial capillaries and cultured skin fibroblasts. These non-neuronal cells appeared to otherwise be cytologically normal, including affected Schwann cells. Hence it appears that although many tissues of the body were sub-clinically affected by this microfilament disorder, peripheral nerve axons, which may be a meter in length, are unusually predisposed to suffer the consequences of such fila-ment accumulations.

### II(C). Diabetic Polyneuropathy and Related Metabolic Symptomology

This section will concern what is known of the biochem-istry of diabetic polyneuropathy and how this pathophysiology appears to serve as a common basis for diabetic complications of other tissues, such as the kidney, the lens, and the vascular system.

Many of the physiological and ultrastructural changes which accompany diabetes are not regarded as phenomena which depend directly on the action of insulin. Rather, they depend on long term hyperglycemia and occur in tissues where glucose uptake is not mediated by insulin. These secondary diabetic phenomena in-clude activation of the polyol pathway and subsequent damage to eye, kidney and nerve tissue; thickening of capillary basement membranes; and non-enzymatic glycation of a diverse array of proteins, including hemoglobin, lipoproteins, albumin, collagens and other basement membrane proteins (Seifter, S and Englard, S, 1990, pg. 1).

Early diabetic peripheral nerve damage can be documented as slowed nerve conduction velocities. Most diabetic patients will show such decreased conduction velocities, and many have or will eventually show additional signs of nerve damage. Autonomic nerves are also affected in diabetic cases. The nerve hyper-trophy seen in diabetic sural nerve samples has been likened to that seen in type I Charcot-Marie-Tooth disease (Johnson, PC, 1985, pg. 334).

Although the specific etiology of diabetic polyneuropathy is still under debate, there is a growing concensus that the axon, not the Schwann cell, is the site of primary etiology (Yamamura, Y et al., 1982, pp. 83-84). Sima (1982) observed that the earliest cytopathological effect seen in sural nerve and spinal ganglion samples from the spontaneously diabetic BB-Wistar rat is the marked- disorientation of axonal neurofilaments. Sidenius and Jakobsen (1982) noted the possibility that reductions in slow axonal transport observed in diabetes may result from re-duced delivery of neurofilaments to distal axonal segments.

### II(C)1. The Polyol Pathway

The clinical onset of diabetic poly-neuropathy has been positively correlated with increased levels of polyol pathway sugars within peripheral neurons in diabetes mellitus and experimental diabetic models. In addition, use of aldose reductase inhibitors has been shown to reduce intraneu-ronal levels of sorbitol and fructose, and a concomitant im-provement in nerve conduction velocities has been observed. Recognized experimental aldose reductase inhibitors include sorbinil (or CP 45,634, Pfizer), tolrestat (or AY 27,773, Ayerst), statil (or ICI 128,436, I.C.I. Ltd.), ONO 2235 (ONO), M 79,175 (Eisai) and AL 1576 (Alcon) (Kinoshita, JH et al., 1990, pg. 269). Hence convincing evidence has been presented which indicates that activation of the polyol pathway is a fundamental part of the etiology of diabetic polyneuropathy. Yet the exact sequence of neurotoxic events remains to be defined. Neither sorbitol nor fructose is neurotoxic per se.

Attempts to define how activation of the polyol pathway initiates neuropathy have focused on two concepts; increased intraneuronal osmotic pressure due to sorbitol and fructose accumulation, and possible depletion of intraneuronal myoinositol, which in turn may limit the activity of Na⁺/K⁺-ATPase. Yet both of these concepts remain unproven. Ward and coworkers (1972) noted that the observed increases in polyol pathway sugars seen in the streptozotocin rat would not be expected to raise nerve osmotic pressure by more than 5 %.

This suggests that osmotic pressure within nerve fibers is not a major etio-logical factor. Published studies on this question in recent years have failed to clearly define an etiological role for myo-inositol. As Clements (1979) noted, studies on autopsy nerve samples from diabetes mellitus patients showed increased levels glucose, sorbitol and fructose, yet normal levels of myo-inositol. Hale and coworkers (1987) examined sugar levels in nerve biopsies from diabetic and non-diabetic patients under-going leg amputation.

They found elevated levels of glucose, sorbitol and fructose in diabetic samples, yet normal levels of nerve myo-inositol.

Thus although activation of the polyol pathway has clearly been linked to onset of diabetic neuropathy, the mechanism by which this occurs has yet to be determined. As discussed below in Section VI (B), it is the belief of this inventor that conversion of fructose to 5-hydroxymethyl furfural and possibly 2,5-furandialdehyde may in fact be the basis of neurotoxic consequences resulting from activation of the polyol pathway.

### II(C)2. Diabetic Non-Enzymatic Protein Glycosylation

Studies during the past decade have clearly established that long-term hyperglycemia leads to generalized non-enzymatic addition of reducing sugar residues to proteins via covalent addition to amine functional groups located on amino acid sidechains. Fol-lowing initial addition, several structural rearrangements occur which can result in intra- and intermolecular crosslinking of proteins. This is a complex series of non-enzymatic reactions which are not completely defined at this time, yet there is reason to believe that this phenomenon is involved in diabetic vascular changes, cataracts and other secondary diabetic symp-tomology. Such reactions may also underlie much of the etiology of aging (Pongor, S et al., 1984).

Public domain information on non-enzymatic glycosylation has re-cently been reviewed (Brownlee, M, 1990). The effects of this phenomenon in long term diabetes are most readily apparent in proteins which have the lowest turnover rates, such as extracel-lular matrix components. Brownlee and others have made a dis-tinction between early diabetic non-enzymatic glycosylation reactions, which are largely reversible by insulin therapy, and advanced non-enzymatic glycosylation products, which are com-plexes of long-lived proteins and sugar-derived crosslinking structures. Brownlee (1990, pg. 282) has described evidence that in the diabetic state advanced glycosylation end (AGE) product-modified low density lipoprotein crosslinks with the collagen of vessel walls, which may underlie the accelerated onset of atherosclerosis seen in diabetic patients. The cross-linking of other circulating proteins such as albumin and im-munoglobulins to vascular walls has also been observed (Brown-lee, M, 1990, pg. 283).

### II(D). Alzheimer's Pro-Senile/Senile Dementia and Down's Syndrome

Definitive diagnosis of Alzheimer's disease (AD) requires histological analysis of a brain biopsy, with pathological find-ings including neurofibrillary tangles, senile plaques, as well as granulovascular and Hirano bodies (Cohan,.SL, 1989, pp. 164-165). Intracellular neurofibrillary tangles and extracellular senile, or neuritic, plaques are the two principle cytological hallmarks of AD. Such Alzheimer type pathological changes are also characteristic of almost all Down's syndrome cases beyond thirty years of age (Goodison, KL et al., 1989).

In AD, histological analysis using silver staining reveals thick bundles of fibrillar material dominating the intracellular en-vironment. In AD and most other disorders featuring neurofib-rillary tangles ultrastructural studies reveal bundles of paired helical filaments (PHF) of a structure not normally seen. Each PHF is a pair of 10 nm filaments wrapped around one another in 80 nm long intervals. PHF's are also characteristic of Guam Parkinsonism-dementia complex, postencephalitic Parkinsonism, dementia pugilistica and adult stage Down's syndrome, and may be present in other neurological disorders.

As gross intracellular neurofibrillary hyperplasia is much more severe in pre-senile AD, while senile plaques are the most distinctive lesion in senile AD (Wisniewski, HM et al., 1982, pp. 110-112), it appears that aberent neurofibrillary proliferation is a relatively early manifestation of a degenerative process which produces senile plaques as its end stage. The senile plaques of AD characteristically feature distension of neural processes, dendrites in this case. Such axonal and/or dendritic distensions, sometimes described as "balloons," have been shown to be present in several neurological disorders. Their ultra-structure typically reveals excess bundles of neurofibrillary tubules (Wisniewski, HM et al., 1970).

Although the point is still under active investigation, presently available evidence indicates that PHF is derived from protein normally present in nerve cells, as opposed to poly-peptides of completely novel origin (Mattson, J and Mattson, MP, 1989; Wisniewski, HM et al., 1982, pg. 116). Immunocytochemical studies have provided evidence for the presence of neurofilament (Elovaara, I et al., 1983; Perry, G et al., 1985; Miller, CC et al., 1986) and neurotubule (Perry, G et al., 1985; Bancher, C et al., 1989) determinants in PHF, as well as determinants for ubi-quitin (Bancher, C et al., 1989) and other proteins, some of which may be fortuitously trapped by altered cytoskeleton com-ponents (Moran, MA and Gomez-Ramos, P, 1989). Analysis of PHF by trypsin or chymotrypsin digestion followed by two dimensional peptide mapping has also provided data which indicate the pre-sence of neurofilament and neurotubule proteins (Iqbal, K et al., 1978). Hence the chemical composition of neurofibrillary tangle PHF is known in some detail.

However, the nature of the chemical bonds responsible for holding neurofibrillary tangles together is still poorly understood (Selkoe, DJ, 1982a). What limited information is publicly available on this question is compatable with the overall premise of this invention; that cytotoxic consequences result from various forms of spurious covalent bond protein crosslinking, at least some forms of which may be clinically treated by the pharmacological procedures described herein.

The occurrence of excess intraneuronal lipofuscin has also been described as part of the histopathology of AD (Sumpter, PQ et al., 1986). Heart lipofuscin has been shown to have the follow-ing general composition: lipids, 20-50%; protein, 30-60%; and strongly pigmented resin-like hydrolysis-resistant material, 9-20%. Although the exact nature of the hydrolysis-resistant chemical bonds remains to be unequivically defined, the similar-ity between lipofuscin fluorescence and that of Schiff bases formed between malondialdehyde and primary amines suggests that similar chemical crosslinks may be part of lipofuscin structure (Tsuchida, M et al., 1987). Histological and ultrastructural changes analogous to those of AD may also be seen in Pick's disease, another central nervous system disorder of the elderly (Yoshimura, N, 1989).

### II(E). Parkinson's Disease (PD)

Several clinically related dis-orders have been described, including postencephalitic parkin-sonism, the Parkinsonism-dementia complex of Guam and juvenile parkinsonism. At the microscopic level Parkinson's disease is most characteristically distinguished by the presence of Lewy bodies, each of which is a concentric hyaline cytoplasmic inclusion consisting of protein filaments densely packed in a central core and more loosely packed in an outer zone (Oppenheimer, DR, 1976, pp. 612-614). Affected neurons progressively accumulate Lewy bodies and eventually die (Marsden, CD, 1983).

Tiller-Borcich and Forno (1988) observed that antibodies to phosphorylated neurofilaments bind to both Lewy bodies of PD and classical Pick bodies, while antibodies to paired helical fila-ments bind only to Pick bodies. PD dementia patients have also been shown to have cortical neurofibrillary tangles and senile plaques similar to those seen in cases of Alzheimer's disease (Cohan, SL, 1989, pg. 167).

As is the case with Alzheimer's disease neurofibrillary tangles, those of the Guam amyotrophic lateral sclerosis-Parkinsonism dementia complex consist of paired helical filaments (Wisniew-ski, HM et al., 1982, pg. 112). In a recent immunochemical study by Shankar and colleagues (1989), the neurofibrillary tangles of the Guam amyotrophic lateral sclerosis-Parkinsonism dementia complex were shown to exhibit "robust" immunoreactivity with antibodies for phosphorylated neurofilaments and paired helical filaments of the Alzheimer type, as well as antibodies for the microtubule-associated protein tau. They also noted that "...many axonal spheroids labeled with the antibody to phosphorylated neurofilament, were observed in the loecular layer of fascia dentata and the stratum radiatum and stratum oriens of Ammon's horn." Tan and coworkers (1981) have also noted increased lipofuscin content in cerebral cortex neurons of Guam Parkinsonism-dementia patients.

### II(F). Amyotrophic Lateral Sclerosis (ALS)

An important clue as to the etiology of ALS was provided by Carpenter in 1968. In examining biopsy material from eleven cases of sporadic ALS he observed the frequent occurrence of large focal axonal spheroids located near nerve perikarya in the spinal cord anterior horns and brainstem motor nuclei. These pathological structures tend-ed to be found in nerve tissues showing relatively early signs of deterioration. Electron microscopic examination showed that these spheroids, or axonal balloons, contained large numbers of neurofilaments. These observations have been confirmed inde-pendently (Chou, SM et al., 1970). Some evidence of increased amounts of neuronal lipofuscin in ALS biopsy material has also appeared (Carpenter, S, 1968; Engel, WK, 1969, pp. 225-227).

### III. BACKGROUND OF THE ART: OTHER DISEASES WHICH MAY BE AMELIORATED BY DRUGS WHICH STABILIZE NEUROFILAMENT METABOLISM

As summarized above, studies suggest that diseases discussed in Section II may feature primary etiology which directly involves spurious, pathological crosslinking of proteins. As summarized below, there are other diseases which show evidence of protein-protein and/or protein-lipid crosslinking which may be part of their respective secondary, if not primary, disease etiologies. Patients experiencing these diseases may also benefit from the drug therapy procedures described herein.

### III(A). Huntington's Disease (HD)

The histopathological study of Tellez-Nagel and coworkers (1974) provides a representative description of HD neuronal cytopathology. Neurons undergoing degeneration demonstrated a variety of intracellular changes which affected lysosomes, Golgi-associated structures, endo-plasmic reticulum, mitochondria, chromatin and nuclear mem-branes. However, they drew particular attention to the pro-gressive appearance of lipofuscin:
...One of the most outstanding features was the large and generalized accumulation of lipofuscin in neurons and glial cells...
...Large accumulation of lipofuscin granules were frequent in the [nerve cell] perikarya, which sometimes resembled storage cells such as those seen in patients with lipidoses.

### II(B). Tinnitus (Nerve Deafness)

Like many other clinical categories described in this text, nerve deafness is actually a syndrome, or, worse yet, a group of syndromes. One part of the nerve deafness complex is Meniere's disease, the symptoms of which include vertigo, tinnitus, and progressive deafness. A number of Meniere's disease clinical sub-varieties have also been recognized (Ylikoski, J et al., 1980). Another part of the nerve deafness complex includes many patients having Charcot-Marie-Tooth syndrome (HMSN I and II). Autosomal recessive CMT has been described in association with deafness (Cornell, J et al., 1984), as have the X-linked (Cowchock, FS et al., 1985) and autosomal dominant (Kousseff, BG et al., 1982) forms. Nerve deafness has also been described in association with a wide variety of other disorders.

Unfortunately, the histopathological research literature on Meniere's disease and related forms of nerve deafness is sparce. The study by Ylikoski and coworkers (1980) on 40 Meniere's dis-ease vestibular nerve biopsy samples is one of the more thorough investigations on this subject. These samples represented long term clinical deterioration, as they were taken from patients undergoing neurectomy operations to treat intractable vertigo. Electron microscopy revealed disorganization of myelin sheaths, evidence of neuronal loss, and evidence of astrocytic gliosis. Neurons showed large numbers of lipofuscin inclusions and variable quantities of neurofilaments, with some cells showing collections of neurofilaments.

### III(C). Spinal Muscular Atrophy

At least three clinical sub-varieties of this syndrome are recognized; infantile- (Werdnig-Hoffmann disease), juvenile- and adult-onset. The findings of Lee and coworkers (1989) may be cited as a recent and detailed cytopathological study of Werdnig-Hoffmann disease. Their patient showed characteristic atrophy and ballooning degeneration of the spinal cord anterior horn cells, most. notably in the lumbosacral segment. Some swollen neurons were also ob-served in Clarke's column, dorsal root ganglia and the cere-bellum. These ballooned neurons were shown to be highly reactive with monoclonal antibody to phosphorylated neuro-filament. In Werdnig-Hoffmann disease this ballooning process occurs in the nerve cell perikaryon. Lee and coworkers noted that normally non-phosphorylated neurofilament is predominantly found in nerve cell bodies, with phosphorylation occurring at points in the perikaryon immediately before neurofilament passes down long axons. They suggested that their findings were in agreement with a previously stated hypothesis that in such patients "...the neurofilaments are abnormally synthesized and phosphorylated in the neuronal perikarya, and/or the axonal transport of phosphorylated neurofilament is impaired, resulting in accumulation in the cell body."

### III(D). Age-Related Atrophy of Peripheral Sensory and Motor Nerves, Autonomic Nerves, and Neurons of the Central Nervous System; and Pathophysiologically Related Changes in the Kidney, Optic Lens and Skin

Bellamy (1988) offered a definition of aging which is relevant to this text: "...the result of somatic damage arising either internally from errors in the operation of biochemical and physiological systems, or externally from the random impact of physical and chemical factors in the environ-ment." As defined below, these age-related changes share much in common with other disease entities discussed in this inven-tion. At the biochemical level, the two most clearly defined pathological events within mammalian cells appear to be (1) the progressive accumulation of lipofuscin and (2) concomitant appearance of high molecular weight protein aggregates and/or polymeric lipid-protein complexes (Shimasaki, H et al., 1984).

Examination of human sural nerve biopsies has revealed age-related degeneration of both myelinated and non-myelinated fibers. This process includes the occurrence of unusual inclusions within axons consisting of filament bundles which appear more dense than those of normal neurofilaments (Ochoa, J and Mair, WG, 1969). The aging brain takes on a progressively more yellow appearance due to lipofuscin deposition in neurons, glia and other cell types (Calne, DB, 1985, pg. 233). Neuronal loss occurs in many areas of the brain. Senile plaques and intraneuronal neurofibrillary tangles, most notably characteristic of Alzheimer's disease, are also seen with increasing frequency in the normal aging brain (Selkoe, DJ et al., 1982). Neuroaxonal dystrophy, characterized by protein-rich swellings of axons, is also a recognized feature of the aging brain (Calne, DB, 1985, pg. 233; Fujisawa, K, 1967).

As peripheral, autonomic and central nervous system neurons lose functional ability as part of the aging process a variety of body functions under their control are adversely affected. Autonomic nervous system functions include urinary continence, peristaltic movement of the digestive tract, sexual response and breathing. Forms of neurological dysfunction lying within the scope of this invention which may cause urinary incontinence include: Alzheimer's senile dementia, demyelinating diseases (e.g., multiple sclerosis), peripheral nerve lesions, diabetes mellitus and alcoholic neuropathy (Palmer, MH, 1985, pg. 27). Drugs which are presently recognized for use in treatment in-clude cholinergics (e.g., bethanechol), anti-cholinergics (e.g., belladonna) and α-adrenergics (e.g., ephedrine) (Palmer, MH, 1985, pg. 58). None of these therapeutic agents have been here-tofore recognized as drugs falling within the pharmacological scope of this invention, although this inventor regards the α-adrenergics ephedrine, which contains a secondary amine group, and phenylpropanolamine, which contains a primary amine group, as potential carbonyl-trapping agents.

Age-onset changes in kidney cell structure share much in common with diabetic changes. In their study of the aging rat, Bolton and Sturgill (1982) observed time-dependent increases in: number of glomular basement membrane (GBM) irregularities; degree of mesangial sclerosis; mesangial expansion into capillary lumina; number of collapsed capillary loops; degenerative cytoplasmic changes in endothelial, mesangial, visceral, parietal epithelial and tubular cells; proteinuria; interstitial mononuclear infil-trates; and thickness of GBM, Bowman's capsule basement membrane and tubular basement membrane.

Senile pathological changes in the optic lens have been observed which are qualitatively similar to those observed in the diabet-ic state. As noted by Creighton and coworkers (1978), the pro-cess of lens fiber cell death has been ascribed to crosslinking or free radical reactions, or possibly gene mutation. Some chemical evidence is now available which supports these ideas. In their study on human senile and diabetic cataracts, Rao and Cotlier (1986) noted evidence that crosslinking of lens proteins via nonenzymatic glycosylation appears to be an underlying pathological mechanism for both cataract types. In their analy-sis of senile cataracts these investigators observed statisti-cally significant decreases in soluble protein content, in-creases in insoluble proteins, decreases in free epsilon-amino groups of insoluble proteins and increases in observed 5-hydroxymethyl furfural levels (i.e., reducible Maillard pro-ducts) in insoluble proteins. Similar data were obtained from diabetic cataracts. Earlier studies showed the appearance of covalently crosslinked protein polymers during senile cataract formation (Selkoe, DJ et al., 1982a).

Evidence of increased lipid peroxidation in the aged human lens has also been presented. Bhuyan and coworkers (1986) noted that concentrations of lens water soluble, non-protein bound thiobar-bituric acid-reactive material remain relatively constant in non-cataractous humans of 11 to 40 years of age, then increase four-fold during 41 to 80 years. This water soluble, carbonyl-containing material includes malondialdehyde as well as sub-stantial amounts of other components not yet identified. These investigators concluded that "...lipid peroxidation of the lens appears to be an age-linked process, enhanced in cataractous lenses."

Changes in skin collagen appear to be a fundamental part of the aging process in this tissue. Long-lived proteins such as collagen, lens crystallins, basement membrane proteins and basic nerve myelin protein have been shown to be more susceptible to non-enzymatic glycosylation (Rao, GN and Cotlier, E, 1986).

Several published studies have presented evidence which implicates lipid peroxidation products in the etiology of atherosclerosis. As summarized by Steinbrecher (1987), there is reason to believe that reactive lipid peroxidation agents form Schiff base adducts with the lysine **epsilon-amino** groups of low density lipoproteins (LDL). Such modified LDL's are recognized by high-affinity acetyl-LDL receptors located on macrophages, which results in lipid accumulation. Lipid-laden macrophages appear to be precursors of the foam cells which populate early atherosclerotic lesions (Stein-brecher, UP, 1987).

### III(E). Friedreich's Ataxia (FA)

Lamarche and coworkers (1982) presented spinal ganglion nerve ultrastructural findings ob-tained from a typical FA case. These investigators observed variable amounts of lipofuscin granules, with some neurons containing great quantities of this substance. However their most pathonometric finding concerned the presence of neurofila-mentous proximal axonal swellings. They noted, in part "...The most striking finding was the presence of numerous axonal swel-lings usually close to the nerve cell body. The axonal swelling consisted mainly of dense accumulation of neurofilaments mea-suring about 10 nm...".

### III(F). Alcoholic Polyneuropathy

Appenzeller and Richardson (1966) conducted a light microscopy study on alcoholic poly-neuropathy sympathetic ganglia samples obtained at autopsy. They observed many degenerating giant neurons in sympathetic ganglia. In some sections as many as 30% of the neurons were of the unusually large variety. These cells were filled with a refractile material of eosinophilic, periodic acid-Schiff reaction positive nature which did not stain with scarlet red or Sudan black B. These histological findings indicate that the refractile material was rich in neurofibrils and carbohydrate but had insignificant amounts of lipid. The material, however, was not further characterized. In the same study these investigators reported similar findings in sympathetic ganglia obtained from patients having diabetes mellitus.

### III(G). Multiple Sclerosis

The results of several published research studies suggest that dysfunctional lipid peroxidation may be a contributing factor in the etiology of multiple sclerosis (Hunter, MI et al.,1985).

### III(H). Juvenile Ceroid-Lipofuscinosis

Juvenile ceroid-lipofus-cinosis cytopathology features prominent accumulation of lipo-fuscin granules in brain nerve cell bodies. In addition, excess lipofuscin accumulation can readily be demonstrated in many other biopsied tissues, including sural nerve, Schwann cells, lymphocytes, macrophages, skin fibroblasts and smooth muscle cells (Schwendemann, G, 1982).

### III(I). Muscular Dystrophy Disorders

Several lines of evidence suggest that the secondary etiology of DMD may include disrup-tion of normal lipid peroxidation homeostasis (Hunter, MI and Mohamed, JB, 1986). Kar and Pearson (1979) have presented evidence of increased glutathione reductase and catalase activities in human DMD muscle samples.

These investigators also reported increased levels of thiobarbituric acid-reacting substances in DMD muscle, an indication of increased presence of lipid peroxidation aldehyde products such as malondialdehyde. This observation has been independently confirmed (Jackson, MJ et al., 1984).

The accumulation of 100 Angstrom (i.e., 10 nm) protein filaments within axonal processes has been observed in infantile neuro-axonal dystrophy. Similar neurofibrillar abnormalities have been observed in axons of IDPN encephalopathy and vitamin E deficiency, in both axons and perikarya of vincristine neuropathy, and in perikarya of sporadic motor neuron disease (Wisniewski, H et al., 1970, pg. 173).

### III(J). Miscellaneous Disorders

The clinical neurology literature includes many descriptions of patients having an incipient form of a disease, patients showing the recognized symptoms of a disease and additional symptomology, and patients demonstrating concurrent clinical symptomology of two or more recognized disease entities. Such clinical disorders are frequently excluded from biochemical studies due to inherent problems of classification and their happenstance occurrence. Hence comparatively little research information is available on such clinical phenomena. Yet it is the understanding of this inventor that information available on the etiologies of well recognized neurological disorders, as summarized herein, can also be extrapolated to infer that the drug therapies described in this text may also be applied with success to the incipient and more complex forms of the diseases described above.

### IV. BACKGROUND OF THE ART: CHEMICAL MODELS OF NEUROFILAMENT ASSOCIATED NEUROPATHIES

Several experimental models of peripheral neuropathies have been described in some detail in the neuropathology literature. These chemical models have provided important opportunities for investigators to study disease etiologies.

The neurotoxicity of chronic hexane exposure has now been repeatedly confirmed in experimental animal studies (Spencer, PS et al., 1980). In 1973 2-hexanone, a hexane oxidation product, was also shown to be the cause of an industrial outbreak of sensorimotor neuropathy. Subsequent animal studies have established that onset of peripheral neuropathy is most closely related to the maximum endogenous concentration of 2,5-hexanedione (2,5-HD), a metabolite of both n-hexane and 2-hexanone (Spencer, PS et al., 1980). 2,5-Hexanediol, 5-hydroxy-2-hexanone and 2-hexanol have also been shown to be significant-ly neurotoxic (Krasavage, WJ et al., 1980).

The cytopathological damage observed in hexacarbon neuropathies has much in common with that of giant axonal neuropathy and the morphological changes seen in alloxan diabetic neuropathy (Powell, HC et al., 1978). At the cellular level, hexacarbon neuropathies induce giant axonal swellings which consist of masses of 10 nm neurofilaments. This has been observed in both human samples (Allen, N et al., 1975) and experimental animals such as rats, chickens and cats (Mendell, JR et al., 1974).

Studies on 2,5-hexanedione neuropathy in rats indicate that this hexacarbon or a derivative of it serves as a protein covalent crosslinking agent (Carden, MJ et al., 1986). Current under-standing of the etiology of hexacarbon neuropathies is that γ-diketones such as 2,5-hexanedione preferentially crosslink the largest neurofilament polypeptide because of its high lysine content. Although the chemical structure(s) of 2,5-hexane-dione-lysine derived crosslinks has still not been explicitly defined, in vitro model studies have suggested that one possible mechanism for crosslink formation may be initial formation of dimethylpyrrole, followed by autoxidation to orange chromophore products and concomitant peptide crosslinking (Graham, DG et al., 1982; Boekelheide, K, 1988).

In addition to the findings summarized above, this inventor will note that some evidence exists which suggests that furanalde-hydes may play a role in hexacarbon neuropathies. Spencer and coworkers (1980, pg. 310) noted earlier studies on shoe factory workers exposed to commercial hexane which indicated that 2,5-dimethylfuran accounted for 32% of the hexane metabolites found in urine samples. Most of the balance was 2,5-hexanedione (36%) and γ-valerolactone (30%). Spencer and coworkers (1980, pg. 304) also noted that 2,5-dimethylfuran is a metabolic product of 5-hydroxy-2-hexanone, along with 2,5-hexanedione and γ-valerolactone.

Williams (1959, pp. 152-153) noted an earlier study by Kuhn and coworkers (1937) which described the metabolic oxidation of 2,5-dimethylfuran to 5-methylfuroic acid. Williams (1959, pp. 545-549) also noted that furfural is converted in vivo to alpha-furoic acid and furylacrylic acid, both of which are recovered in part as their glycine conjugates, and that methylfuran is oxidized to α-furoic acid. The formation of furylacrylic acid may be the result of a Perkin synthesis with acetic acid. Williams (1959, pp. 550-551) also mentioned the metabolic con-version of 5-hydroxymethyl furfural, described as a well-known product of the action of acids on hexose sugars, to 5-hydroxy-methyl-2-furoic acid. Hence furan derivatives such as 2,5-dimethylfuran may participate in a variety of in vivo pro-gressive oxidation steps.

As the occurrence of genetic peripheral motor and sensory neuro-pathy has recently been linked to defective furancarboxylic acid excretion (Shapiro, HK et al., 1986; Shapiro, HK and Kahn, GC, 1990; see Section II [A] of this invention), the prospect that furanaldehydes may play a role in hexacarbon neuropathy may warrent additional consideration.

A variety of additional chemically induced human and/or experimental animal models of peripheral sensorimotor, autonomic and/or central nervous system neuropathies have been described in the public domain biomedical literature (Osuntokun, BO, 1982). As for the hexacarbon neuropathies, published studies of many of these additional chemical models have revealed evidence of pathological protein crosslinking and/or lipofuscin accumul-ation. These include several well documented animal models of diabetes. Also included in this catagory are peripheral sen-sorimotor neuropathies induced by vincristine sulfate or vin-blastine sulfate (Shelanski, ML and Wisniewski, H, 1969), peripheral sensorimotor neuropathy induced by diamminodichloro-platinum (Kaplan, RS and Wiernik, PH, 1982), doxorubicin peri-pheral neuropathy (Parhad, IM et al., 1984; Ogura, R, 1982), β,β'-iminodiproprionitrile neuropathy (Smith, WT, 1976, pg. 225), progressive neuropathy due to vitamin E deficiency (Diplock, AT, 1984; Wisniewski, H et al., 1970; Lampert, P et al., 1964; Derrick, NM and Wishner, LA, 1967; and Miyagishi, T et al., 1967), acrylamide neuropathy (Gold, BG, 1987; Davenport, JG et al., 1976), tri-orthocresyl phosphate peripheral neuro-pathy (Prineas, JW, 1969, pg. 582) and carbon disulfide-induced polyneuropathy (Juntunen, J et al., 1974, pg. 363).

### V. OBJECTS OF THE INVENTION

Accordingly, it is a general object of this invention to treat neurological diseases and etiologically related symptomology by use of primary agents so as to overcome the disad-vantages of the prior art.

### V(A). Section II Disorders

In particular, it is an object of the present invention that the drug compounds described herein may be of clinical value in treatment of disease symptomology for disorders featuring well defined neurofilament associated pathology, including: hereditary motor and sensory neuropathies; giant axonal neuropathy; diabetic polyneuropathy and related metabolic symptomology; Alzheimer's presenile/senile dementia; Down's syndrome; Pick's disease; Parkinson's disease; amyo-trophic lateral sclerosis; and disorders clinically related to those listed above.

### V(B). Section III Disorders

It is a further object of the present invention that the drug compounds described herein may be of clinical value in treatment of disease symptomology for neurological disorders featuring axon deterioration as defined in Section III of this invention, including: Huntington's disease; tinnitus (nerve deafness); spinal muscular atrophy; age-related atrophy of peripheral sensory and motor nerves, autonomic nerves, and neurons of the central nervous system as well as pathophysiologically related changes in the cardiovascular system, kidney and optic lens; Friedreich's ataxia; alcoholic polyneuropathy; multiple sclerosis; ceroid lipofuscinosis; muscular dystrophy disorders; and miscellaneous disorders as defined in Section III(J).

### V(D). Treatment of Autonomic Disorders

It is another object of the present invention that in so far as the therapeutic pro-cedures described herein may be of benefit for improvements in autonomic nervous system function, it is claimed that such pro-cedures may ameliorate symptomology of hypoperistalisis of the alimentary tract; hiatal hernia and partial food regurgitation; urinary incontinence; breathing insufficiency due to diaphram weakness and decreased autonomic sexual function.

### V(E). Treatment of Atherosclerosis Symptomology

It is yet another object of the present invention that in so gfar as the therapeutic procedures described herein may serve to covalently bind and sequester agents which may underlie, in part, the etiology of atherosclerosis, it is believed that such procedures may be of benefit in treatment of this disorder.

### V(F). Tablet Composition

In so far as the primary amine substances described above may be applied to treatment of the clinical disorders summarized in Sections V(A), V(B), V(C), V(D) and V(E) of this text, it is claimed that they may be of clinical value when applied under the following conditions. Tablet composition may optionally include a suspending reagent selected from the group consisting of carboxymethyl cellulose or functional equivalents thereof.

### V(F)1. Primary Agent Dosage

The primary agents are believed to be of value in reducing endogenous concentrations of carbonyl substances when administered orally within a dosage range of 600 mg/day to 40 gm/day.

At least one of the following agent is combined to the primary agent.

### V(F)2. Co-Administration of Antioxidants

It is claimed that the therapeutic value of primary agents described herein may be maximized by administration in conjunction with recognized free radical trapping compounds such as vitamin E (Stuckey, BN, 1968, pp. 214-215) or other co-agents previously recognized as adjunts which facilitate in vivo capability to inhibit lipid peroxidation, such as selenium (Stuckey, BN, 1968, pg. 236). Citric acid may also be included in this catagory of co-administered agents, as it is recognized as having antioxidant properties (Merck Index, 11th edition, pg. 363). This agent is also recognized as an inhibitor of Maillard reactions (Stuckey, BN, 1968, pg. 210). In a published list of substances which function to supplement the chain-breaking antioxidant property of vitamin E, Tappel (1970, pg. 1138) mentioned ubiquinol, seleno-amino acids and sulfhydryl compounds (e.g., glutathione, sulfhydryl proteins, cysteine and methionine). Ascorbic acid is not included in this catagory, as published studies indicate that it may function as a pro-oxidant (Ballin, A et al., 1988, pg.. 119), may initiate lipid peroxidation (Chojkier, M et al, 1989, pgs. 16957 and 16961), and may readily glycosylate proteins (Slight, SH et al., 1990).

### V(F)3. Hormone Co-Administration

It is another object of this invention that in so far as the primary agents described herein may be applied to the treatment of age-onset pathological phenomena, it is believed that the therapeutic value of these products may be maximized by administration in conjunction with human growth hormone.

### V(F)4. Prophylactic Vitamin Co-Administration

It is yet still another object of this invention that the safety and effectiveness of the products described herein may be optimized by co-administration of vitamins which may be inadvertently de-pleted by the treatment, such as vitamins A, D and K (depleted by cholestyramine use) or vitamin B₆. Pyridoxal, a biologically active metabolite of vitamin B₆, has an aldehyde functional group in its structure.

### V(F)5. Co-Administration of a Chemical Conjugating Substance

It is another object of this invention that the safety and effectiveness of the products described herein may be optimized by co-administration of glycine as a chemical conjugating substance, as it may be depleted within the body during long term use of the drug compositions disclosed herein.

### V(F)6. Co-Administration of a Metabolite at Risk of Depletion

It is another object of this invention that the safety and effectiveness of the products described herein may be optimized by co-administration of pantothenic acid, which may be depleted within the body during long term use of the drug compositions disclosed herein.

### V(F)7. Co-Administration of Sulfhydryl Containing Substances

Noting the well documented ability of carbonyl containing substances to react with sulfhydryl groups (Jellum, E et al., 1973), it is a further object of this invention that methionine, cysteine, homocysteine and α-lipoic acid may also be of clinical benefit as absorbable drugs capable of covalently binding aldehyde or ketone agents. It is also claimed that these drugs can be used most effectively when administered in conjunction with the primary agents described herein.

### VI(F)8. Factors Affecting Dialy Dosage Schedule

It is claimed that a daily protocol of amine drug composition consumption may be defined such that drug products are administered in timed-release and/or color coded tablets or capsules, so as to maximize therapeutic value and facilitate patient compliance.

### VI. DETAILED DESCRIPTION OF THE PREFERRED EMBODIMENT: DRUG PRODUCTS AND PROPOSED MECHANISM OF PHARMACEUTICAL ACTION OF THE PRESENT INVENTION

### VI (A). Introduction

The inventive feature disclosed in this text is that the absorbable carbonyl-trapping drugs which are the primary agents of this invention may be of use in preventing or ameliorating protein and/or lipid crosslinking reactions which appear to underlie the etiology of many neurological diseases and age-related pathological changes. In this section the inventor summarizes his present understanding of etiological events which have not been publicly recognized up to this point and presents in detail proposed pharmacological procedures for therapeutic intervention.

### VI (B). Description of the Glucose-Fructose-2,5-Furandicarboxylic Acid Pathway

Although not frequently cited for their toxic properties, furan compounds have been identified in a wide vari-ety of food products (Lever, M et al., 1985; Dunlop, AP and Peters, FN, 1953, pgs. 213, 308 and 403; Shimizu, J and Watan-abe, M, 1979; Rice, EW, 1972; Baltes, W, 1985; Pettersen, JE and Jellum, E, 1972). Correspondingly, oxidized furan products such as 2,5-furandicarboxylic acid have been identified in normal human urine samples (Williams, RT, 1959, pp. 551-552; Chalmers, RA and Lawson, AM, 1982, pgs. 164 and 180; Lawson, AM et al., 1976, pg. 1286; Pettersen, JE and Jellum, E, 1972; Pinkston, D et al., 1981; Shapiro, HK and Kahn, GC, 1990). The metabolic origins of 2,5-furandicarboxylic acid are, however, by no means as simple as originally assumed. For example, the metabolic studies of Jellum and coworkers (1973) on two infants clearly established that the dicarboxylic acid is, in fact, generated in vivo from fructose-glucose precursors.

Although few studies have addressed the issue of toxicity of oxidized furans, some published information is available. These reports include toxicity studies on furfural (Konecki, J et al., 1974), 2-furfurol (Dunlop, AP and Peters, FN, 1953, pg. 719) and 5-hydroxymethyl furfural (Ulbricht, RJ et al., 1984).

Beyond inferences to be derived from the findings of this inventor, contained herein, it appears that no one has raised questions regarding the possible toxicity of 2,5-funandialdehyde.

By analogy to one of the proposed reactions of 2,5-hexanedione with amino groups of proteins (Graham, DG and Abou-Donia, MB, 1980, pg. 628), one may envision the di-Schiff base protein crosslink formed from a 5-hydroxymethyl furfural or 2,5-furandialdehyde precursor to have the following structure:

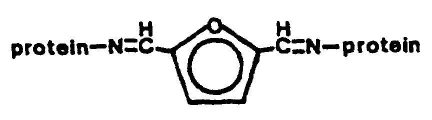

### VI(B)1. Non-Enzymatic Formation and Reactions of Furans

The chemical formation of furans from hexose or pentose sugars is a well recognized reaction which has been applied on an industrial scale for several decades (Dunlop, AP and Peters, FN, 1953, pg. 738). The mechanism of this class of reactions, starting with one of several pentoses or hexoses and ending with furfural or 5-hydroxymethyl furfural, respectively, appears to involve three consecutive dehydration steps (Dunlop, AP and Peters, FN, 1953, pp. 289-290). The ease with which this reaction proceeds is such that the presence of acid is not even required (Dunlop, AP and Peters, FN, 1953, pgs. 281 and 293; Scallet, BL and Gard-ner, JH, 1945). Murty and coworkers (1977) reported that freshly prepared 10% fructose solution contained 0.2. ug 5-hydroxy-methyl furfural per ml, while a commercially available 10% fructose intravenous feeding solution contained 4.2 ug/ml.

Jellum and coworkers (1973) demonstrated that commercially prepared mixed fructose/glucose solutions used for intravenous feeding may contain as much as 1.2 gm 5-hydroxymethyl furfural per liter if autoclaved at pH 2.0. By use of gas chromatography/mass spectrometry they also demonstrated the in vivo oxidation of 5-hydroxymethyl furfural to 5-hydroxymethyl-2-furoic acid and 2,5-furandicarboxylic acid in two human new-borns. Referring to the furans which were not excreted in the babies' urine, Jellum and coworkers noted that
The remaining part of the [5-hydroxymethyl furfural] aldehyde and [all of] the 2-(2'-hydroxyacetyl)-furan are probably bound to thiol and amino groups of proteins and enzymes. The furan derivatives present in sterile fructose-containing solutions may consequently cause harmful effects when infused intravenously in humans.

The tendency of fructose to generate furanaldehyde products more readily than glucose is directly based on the chemical nature of the sugars (Dunlop, AP and Peters, FN, 1953, pp. 405-406; Murty, BS et al., 1977).

### VI (B)2. Reactive Carbonyl Species as Possible Initiators of Disease Etiology

Much of the above discussion has focused explicitly or implicitly on reactions involving covalent addition of aldehydes to side chain amino groups of proteins or amino groups of phospholipids. As summarized in Sections II and III herein, there are reasons to believe that pathological, covalently crosslinked complexes of proteins and/or amine-containing lipids constitute part of the etiological processes which under-lie a number of human neurological diseases. Yet in many disorders such intracellular and extracellular ultrastructural complexes may be secondary etiological phenomena, each being the consequence of some earlier pathological event.

In addition, there are reasons to believe that reactive aldehyde or ketone compounds may on occasion be involved in the primary etiology of a disease. There are at least two particular physiochemical mechanisms by which this might occur.

The reactive carbonyl specie(s) may preferentially bind to one or more proteins having enzymatic activity. Alternatively, the reactive carbonyl specie(s) may preferentially bind to a structural protein, or class of structural proteins. These two possibil-ities may be envisioned in somewhat more specific terms, as discussed below.

In his discussion of age-related changes in the activities of microsomal mixed function oxidase (MFOS) drug metabolizing enzymes, Schmucker (1985) noted data which
...support the concept that 'altered' enzyme molecules characterized by reduced catalytic activity, altered heat inactivation profile, unchanged antigenic cross-reactivity, and essentially unchanged kinetic properties constitute a portion of the microsomal NADPH cytochrome c (P-450) reductase pool in the livers of old rats.

Although Schmucker did not address the chemical basis of such changes in enzymatic activities, the work of Davidson and Flynn (1979) could serve to offer one possible explanation. These investigators studied the high-Kₘ isoform of NADPH-dependent aldehyde reductase of pig kidney. A physiologically similar enzyme, the low-Kₘ adlehyde reductase of mammalian brain, is one and the same as, or a close biochemical isoform of, the aldose reductase of the polyol pathway (Flynn, TG, 1982). Davidson and Flynn have shown that the high-Kₘ isoform of this enzyme includes two essential amino acid residues which contain side chain amino groups, one arginine and one lysine. Both of these key residues appear to be at or near the binding site for NADPH. The investigators demonstrated that the aldehyde reductase is inactivated by 2,3-butanedione, phenylglyoxal, methylglyoxal or 1,2-cyclohexanedione. 2,3-Butanedione was shown to preferen-tially bind one arginine residue per protein molecule. Hence, although the enzyme is biologically active in catalyzing the conversion of many carbonyl-containing substrates (including phenylglyoxal and methylglyoxal) to corresponding alcohols, some carbonyl-containing species can actually bind at or near the enzyme cofactor binding site so as to inactivate it. Aldose reductase and carbonyl reductase, the two other members of this class of enzymes, have also been examined for the presence of an essential arginine residue. Such a peptide residue is present in the carbonyl reductase, but not in the aldose reductase (Boh-ren, KM et al., 1987).

This inventor recognizes the possibility that one or more sugar-derived furanaldehyde compounds may actually be involved in the primary, as well as the secondary, etiology of diabetes. Put another way, the primary etiology of human diabetes may involve chronically increased amounts of furanaldehydes from a high sugar diet leading to inactivation of the high Kₘ aldehyde reductase and/or the carbonyl reductase, with sparing of the aldose reductase. Activation of the polyol pathway, involving aldose reductase, would then only serve to make matters worse.

Interference with the normal activity, or role, of a structural protein might also be a primary etiological event. The normal process of beta cell insulin granule release has been reported to depend on the microtubular system (Pipeleers, DG et al., 1976), which has tubulin as its major protein component. As methylglyoxal and several other aldehydes have been shown to actively bind tubulin (Dianzani, MU, 1978, pg. 253), some in vivo form of such protein binding may be an early diabetogenic event. Dianzani reported, for example, that in vitro colchicine binding to tubulin was inhibited 93.4% by 10 mM methylglyoxal. This certainly raises a question as to the ability of sugar-derived furanaldehydes to bind to tubulin, an issue which has not yet been investigated. Aldehyde binding to beta cell tub-ulin may initiate a diabetogenic sequence featuring decreased insulin secretion; elevated levels of blood and tissue glucose; consequent increases in furanaldehydes; activation of the polyol pathway; increased protein crosslinking and onset of secondary diabetic symptomology, including accelerated demise of pancreatic β cells.

As regards this scenerio, the reader is reminded that (1) mild hyperglycemia and diabetic cataracts develop in the degu, a South American rodent, when captured wild animals are simply maintained on ordinary laboratory rat chow (Varma, SD et al., 1977); (2) experimental diabetic symptomology such as glomerulosclerosis and retinopathy can be induced by high sugar, especially high fructose, diets in the absence of diabetogenic agents such as alloxan (Boot-Handford, RP and Heath, H, 1981); and (3) normal adult humans accustomed to diets low in mono/disaccharide sugars (Yemani immigrants to Isreal) can be inadvertently induced into a diabetic state merely by switching to diets having sugar levels found commonly in the western world (Rosenbaum, E et al., 1971).

### VI(B)3. Summary of Inventor's Understanding of Polyol Pathway

Currently the relationship between activation of the polyol pathway arid onset of secondary diabetic symptomology is well established in both man and experimental animal models (Flynn, TG, 1982). Yet glucose, sorbitol and fructose are not chemically toxic per se.

It is the preliminary conclusion and understanding of this inventor, not previously recognized by other investigators, that the toxic consequences of polyol pathway activation are the result of increased production of 5-hydroxymethyl furfural and 2,5-furandialdehyde brought on by the initial shift to excess endogenous levels of fructose. As fructose does not readily diffuse out of the tissues prone to secondary diabetic symptomology, the effect would be to generate excess levels of furanal-dehydes at these sites. It appears that in the uncontrolled diabetic state, and to a lesser extent in the controlled diabet-ic state, that hyperglycemic endogenous generation of furanalde-hydes exceeds the body's capacity to either reduce these pro-ducts to alcohols (which themselves may be toxic) via aldehyde/ ketone reductases or oxidize them to carboxylic acids via alde-hyde dehydrogenases.

Food chemists confirmed long ago that in Maillard reaction systems, also known as non-enzymatic glycosylation of primary amines, that Amadori products rearrange to form Schiff base-bound 5-hydroxymethyl furfural, and this in turn exchanges with free 5-hydroxymethyl furfural (Keeney, M and Bassette, R, 1959). This is not an isolated observation. Model Maillard reaction studies on difructose-glycine have also demonstrated the generation of free 5-hydroxymethyl furfural (Gottschalk, A, 1972). Mevissen and Baltes (1983) used gas chromatography/mass spectrometry to analzye volatile products generated by the Maillard-type reaction of glucose with phenylalanine. Thirteen of the 29 identified volatile products were furan derivatives, including 2,5-furandialdehyde, 5-hydroxymethyl furfural and 2-furaldehyde. When we compare these empirical observations to the non-enzymatic glycosylation scheme of Brownlee (1990, pg. 281) we see that recognition of furan formation has simply been deleted, a conceptual oversight common in recent studies on the relationship of non-enzymatic glycosylation to diabetes.

A re-evaluation of some of the recently reported data on advanced glycosylation end products may now be offered. In his recent review on this subject Brownlee (1990) did not specifically describe the metabolic origin of the crosslink illustrated below. He suggested that it might originate by an as yet undefined conjugation process involving two Amadori groups and two protein amine groups. This inventor notes the possibility of an alternative metabolic origin. In their paper on the human metabolic origins of furancarboxylic acids Jellum and coworkers (1973) reported detection of 2-(2'-hydroxyacetyl)-furan (see below) in parenteral feeding solutions autoclaved at pH 2.0, as well as 5-hydroxymethyl-2-furfural, levulinic acid and 2-keto-3-deoxyglucose. Jellum and coworkers noted that
It should be borne in mind that aldehydes in general are reactive compounds capable of interacting with thiol and amino groups of proteins. Because of these properties aldehydes may block SH groups essential for cell division, and thus act as cytotoxic agents. Ketoaldehydes are even more reactive mitotic inhibitors. In this connection particular attention should be paid to the keto alcohol 2-(2'-hydroxyacetyl)-furan which according to our results may be present in fructose-containing solutions. This keto alcohol will undoubtedly be oxidised in the human body to the corresponding keto aldehyde which immediately will interact with thiol groups of the cells, and thus possibly cause unwanted effects. It is interesting to note that not even a trace of unchanged 2-(2'-hydroxyacetyl)-furan or any of its likely metabolites could be detected in the urine of the patients. This indicates that the compound had been completely retained in their bodies.

It seems apparent to this inventor that a condensation process involving two molecules of 2-(2'-hydroxyacetyl)-furan and two protein amino groups may also be a metabolic basis of the AGE product crosslink illustrated by Brownlee (1990).

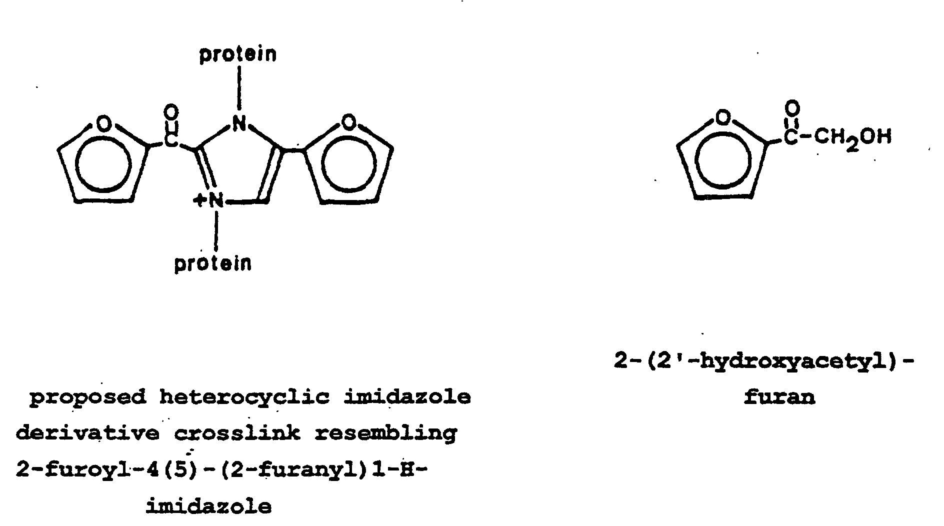

In addition to publicly available information, some unpublished findings of this inventor lend further credence to the conclusion that sugar-derived furanaldehydes play a role in the eti-ology of diabetes. As part of the urine organic acid metabolic screening study on Charcot-Marie-Tooth syndrome patients con-ducted by this inventor (Shapiro, HK and Kahn, GC, 1990) a urine sample from a recently diagnosed adult onset diabetes patient was also examined. At time of sampling the male patient was 59 years of age and undergoing periodic blood testing subsequent to a coronary bypass operation conducted six months earlier. Starting at four months after the operation the patient began showing excess levels of blood sugar and failed a glucose tolerance test. He was advised of his newly acquired status as a diabetic patient, informed as to how dietary sugar comsumption should be limited henceforth, instructed in the use of a paper indicator product for monitoring urine glucose levels, but not pharmacologically treated for his diabetes prior to urine sam-pling for the metabolic screening study. The patient was put on daily insulin therapy approximately two years later, yet diabet-ic complications including confirmed peripheral nerve deficit continued to develop. The patient died of diabetic complications eight years after urine sampling for the metabolic screening study. Levels of urine furancarboxylic acids observed in five normal adult donors for this study and the one newly diagnosed diabetic patient are summarized below. It can be seen from these data that the diabetic patient showed an apparent urine concentration of 5-hydroxymethyl-2-furoic acid which was

| urine donors | 5-hydroxymethyl-2-furoic acid* | 2,5-furandicarboxylic acid* | 5-carboxy-2-furoylglycine* |
|---|---|---|---|
| control-1 | 2.31 | 23.18 | 3.56 |
| -2 | 0.76 | 21.22 | 3.97 |
| -3 | 0.75 | 11.22 | 1.71 |
| -4 | 0.00 | 1.76 | 0.51 |
| -5 | 0.99 | 23.17 | 3.49 |
| diabetic | 24.08 | 113.87 | 17.89 |

| | | | |
|---|---|---|---|
| *measured as percent area relative to internal standard | | | |

approximately 25 times the concentrations observed for normal donors, while his observed levels of 2,5-furandicarboxylic acid or its mono-glycine derivative were approximately six to eight times higher than corresponding control values.

These observations cannot be explained based on present publicly defined understanding of polyol pathway metabolism or formation of non-enzymatically initiated protein glycosylation reactions. Rather, these findings suggest that this diabetic patient was forming excess in vivo concentrations of furanaldehyde precursors, at least some of which were oxidized and excreted in his urine.

### VI (C) . Description of Lipid Peroxidation-2,5-Furandicarboxylic Acid Pathway

The thought that products of lipid peroxidation might include metabolites such as 5-hydroxymethyl-furanaldehyde and 2,5-furandialdehyde has attracted little, if any, attention within the biomedical research community up to this point. As will be described below, 2,5-dimethyl-furan appears to be a key intermediate in this pathway.

### VI(C)1. An Overview of Lipid Peroxidation

Kikugawa and Beppu (1987) have summarized present knowledge of lipid biological peroxidation, including the generation of carbonyl compounds and furans. They also noted that lipid radicals, hydroperoxides and their secondary products react with neighboring protein molecules, damaging protein structure and function. Such damage includes formation of fluorescent chromophores, lipid-protein adducts, and protein-protein crosslinks. Using SDS-polyacryl-amide gel electrophoresis, these investigators demonstrated that malonaldehyde (also known as malondialdehyde), a bifunctional molecule having two aldehyde groups, can covalently crosslink proteins. This reaction primarily involves Schiff base form-ation with protein ε-amino groups on the sidechains of lysine residues. It is now understood that the proposed role of malondialdehyde as the primary aldehyde product of lipid perox-idation has been overstated by many investigators (Vaca, CE et al., 1988; Halliwell, B, 1984).

Gutteridge and Stocks (1976) noted that the formation of lipofuscin-type fluorescent pigments, which normally occurs slowly with aging, can be induced prematurely in experimental animals by oxygen, pro-oxidant reagents such as iron, or exposure to X-rays. The conceptual similarities between lipid peroxidationinduced protein crosslinking and protein crosslinking associated with non-enzymatic glycosylation has been noted in key residues appear to be at or near the binding site for NADPH. The investigators demonstrated that the aldehyde reductase is inactivated by 2,3-butanedione, phenylglyoxal, methylglyoxal or 1,2-cyclohexanedione. 2,3-Butanedione was shown to preferentially bind one arginine residue per protein molecule. Hence, although the enzyme is biologically active in catalyzing the conversion of many carbonyl-containing substrates (including phenylglyoxal and methylglyoxal) to corresponding alcohols, some carbonyl-containing species can actually bind at or near the enzyme cofactor binding site so as to inactivate it. Aldose reductase and carbonyl reductase, the two other members of this class of enzymes, have also been examined for the presence of an essential arginine residue. Such a peptide residue is present in the carbonyl reductase, but not in the aldose reductase (Bohren, KM et al., 1987).

This inventor recognizes the possibility that one or more sugar-derived furanaldehyde compounds may actually be involved in the primary, as well as the secondary, etiology of diabetes. Put another way, the primary etiology of human diabetes may involve chronically increased amounts of furanaldehydes from a high sugar diet leading to inactivation of the high Kₘ aldehyde reductase and/or the carbonyl reductase, with sparing of the aldose reductase. Activation of the polyol pathway, involving aldose reductase, would then only serve to make matters worse.

Interference with the normal activity, or role, of a structural protein might also be a primary etiological event. The normal process of beta cell insulin granule release has been reported to depend on the microtubular system (Pipeleers, DG et al., 1976), which has tubulin as its major protein component. As methylglyoxal and several other aldehydes have been shown to actively bind tubulin (Dianzani, MU, 1978. pg. 253). some in vivo form of such protein binding may be an early diabetogenic event. Dianzani reported, for example, that in vitro colchicine binding to tubulin was inhibited 93.4% by 10 mM methylglyoxal. This certainly raises a question as to the ability of sugar-derived furanaldehydes to bind to tubulin, an issue which has not yet been investigated. Aldehyde binding to beta cell tubulin may initiate a diabetogenic sequence featuring decreased insulin secretion; elevated levels of blood and tissue glucose; consequent increases in furanaldehydes; activation of the polyol pathway; increased protein crosslinking and onset of secondary diabetic symptomology, including accelerated demise of pancreatic β is cells.

As regards this scenerio, the reader is reminded that (1) mild hyperglycemia and diabetic cataracts develop in the degu, a South American rodent, when captured wild animals are simply maintained on ordinary laboratory rat chow (Varma, SD et al., 1977); (2) experimental diabetic symptomology such as glomerulosclerosis and retinopathy can be induced by high sugar, especially high fructose, diets in the absence of diabetogenic agents such as alloxan (Boot-Handford, RP and Heath, H, 1981); and (3) normal adult humans accustomed to diets low in mono/disaccharide sugars (Yemani immigrants to Isreal) can be inadvertently induced into a diabetic state merely by switching to diets having sugar levels found commonly in the western world (Rosenbaum, E et al., 1971).

VI(B)3. Summary of Inventor's Understanding of Polyol Pathway Currently the relationship between activation of the polyol pathway and onset of secondary diabetic symptomology is well established in both man and experimental animal models (Flynn, TG, 1982). Yet glucose, sorbitol and fructose are not chemically toxic per se.

It is the preliminary conclusion and understanding of this inventor, not previously recognized by other investigators, that the toxic consequences of polyol pathway activation are the result of increased production of 5-hydroxymethyl furfural and 2,5-turandialdehyde brought on by the initial shift to excess endogenous levels of fructose. As fructose does not readily diffuse out of the tissues prone to secondary diabetic symptomology, the effect would be to generate excess levels of furanaldehydes at these sites. It appears that in the uncontrolled diabetic state, and to a lesser extent in the controlled diabetic state, that hyperglycemic endogenous generation of furanaldehydes exceeds the body's capacity to either reduce these products to alcohols (which themselves may be toxic) via aldehyde/ketone reductases or oxidize them to carboxylic acids via aldehyde dehydrogenases.

Food chemists confirmed long ago that in Maillard reaction systems, also known as non-enzymatic glycosylation of primary amines, that Amadori products rearrange to form Schiff base-bound 5-hydroxymethyl furfural, and this in turn exchanges with free 5-hydroxymethyl furfural (Keeney, M and Bassette, R, 1959). This is not an isolated observation. Model Maillard reaction studies on difructose-glycine have also demonstrated the generation of free 5-hydroxymethyl furfural (Gottschalk, A, 1972). Mevissen and Baltes (1983) used gas chromatography/mass spectrometry to analzye volatile products generated by the Maillard-type reaction of glucose with phenylalanine. Thirteen of the 29 identified volatile products were furan derivatives, including 2,5-furandialdehyde, 5-hydroxymethyl furfural and 2-furaldehyde. When we compare these empirical observations to the non-enzymatic glycosylation scheme of Brownlee (1990, pg. 281) we see that recognition of furan formation has simply been deleted, a conceptual oversight common in recent studies on the relationship of non-enzymatic glycosylation to diabetes.

A re-evaluation of some of the recently reported data on advanced glycosylation end products may now be offered. In his recent review on this subject Brownlee (1990) did not specifically describe the metabolic origin of the crosslink illustrated below. He suggested that it might originate by an as yet undefined conjugation process involving two Amadori groups and two protein amine groups. This inventor notes the possibility of an alternative metabolic origin. In their paper on the human metabolic origins of furancarboxylic acids Jellum and coworkers (1973) reported detection of 2-(2'-hydroxyacetyl)-furan (see below) in parenteral feeding solutions autoclaved at pH 2.0, as well as 5-hydroxymethyl-2-furfural, levulinic acid and 2-keto-3-deoxyglucose. Jellum and coworkers noted that
It should be borne in mind that aldehydes in general are reactive compounds capable of interacting with thiol and amino groups of proteins. Because of these properties aldehydes may block SH groups essential for cell division, and thus act as cytotoxic agents. Ketoaldehydes are even more reactive mitotic inhibitors. In this connection particular attention should be paid to the keto alcohol 2-(2'-hydroxyacetyl)-furan which according to our results may be present in fructose-containing solutions. This keto alcohol will undoubtedly be oxidised in the human body to the corresponding keto aldehyde which immediately will interact with thiol groups of the cells, and thus possibly cause unwanted effects. It is interesting to note that not even a trace of unchanged 2-(2'-hydroxyacetyl)-furan or any of its likely metabolites could be detected in the urine of the patients. This indicates that the compound had been completely retained in their bodies.

It seems apparent to this inventor that a condensation process involving two molecules of 2-(2'-hydroxyacetyl)-furan and two protein amino groups may also be a metabolic basis of the AGE product crosslink illustrated by Brownlee (1990).

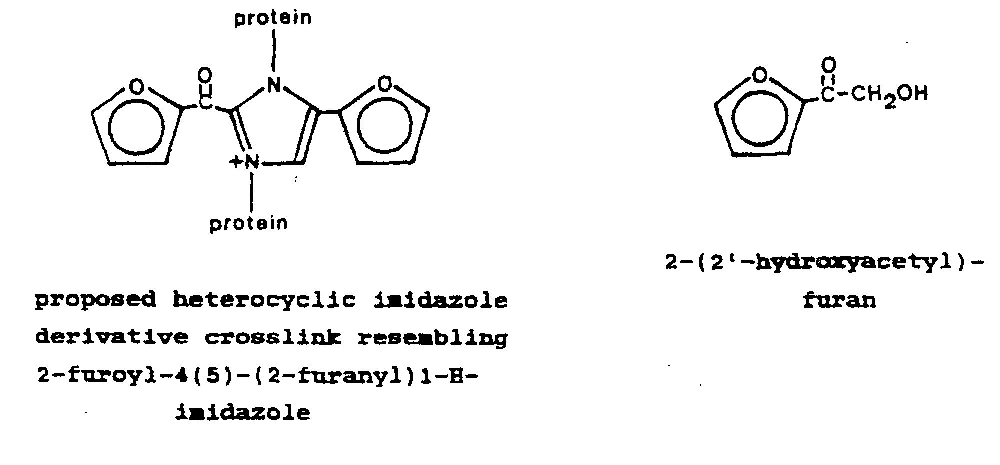

In addition to publicly available information, some unpublished findings of this inventor lend further credence to the conclusion that sugar-derived furanaldehydes play a role in the etiology of diabetes. As part of the urine organic acid metabolic screening study on Charcot-Marie-Tooth syndrome patients conducted by this inventor (Shapiro, HK and Kahn, GC, 1990) a urine sample from a recently diagnosed adult onset diabetes patient was also examined. At time of sampling the male patient was 59 years of age and undergoing periodic blood testing subsequent to a coronary bypass operation conducted six months earlier. Starting at four months after the operation the patient began showing excess levels of blood sugar and failed a glucose tolerance test. He was advised of his newly acquired status as a diabetic patient, informed as to how dietary sugar comsumption should be limited henceforth, instructed in the use of a paper indicator product for monitoring urine glucose levels, but not pharmacologically treated for his diabetes prior to urine sampling for the metabolic screening study. The patient was put on daily insulin therapy approximately two years later, yet diabetic complications including confirmed peripheral nerve deficit continued to develop. The patient died of diabetic complications eight years after urine sampling for the metabolic screening study. Levels of urine furancarboxylic acids observed in five normal adult donors for this study and the one newly diagnosed diabetic patient are summarized below. It can be seen from these data that the diabetic patient showed an apparent urine concentration of 5-hydroxymethyl-2-furoic acid which was

| urine donors | 5-hydroxymethyl-2-furoic acid* | 2,5-furandicarboxylic acid* | 5-carboxy-2-furoylglycine* |
|---|---|---|---|
| control-1 | 2.31 | 23.18 | 3.56 |
| -2 | 0.76 | 21.22 | 3.97 |
| -3 | 0.75 | 11.22 | 1.71 |
| -4 | 0.00 | 1.76 | 0.51 |
| -5 | 0.99 | 23.17 | 3.49 |
| diabetic | 24.08 | 113.87 | 17.89 |

| | | | |
|---|---|---|---|
| *measured as percent area relative to internal standard | | | |

approximately 25 times the concentrations observed for normal donors, while his observed levels of 2,5-furandicarboxylic acid or its mono-glycine derivative were approximately six to eight times higher than corresponding control values.

These observations cannot be explained based on present publicly defined understanding of polyol pathway metabolism or formation of non-enzymatically initiated protein glycosylation reactions. Rather, these findings suggest that this diabetic patient was forming excess in vivo concentrations of furanaldehyde precursors, at least some of which were oxidized and excreted in his urine.

VI(C). Description of Lipid Peroxidation-2,5-Furandicarboxylic Acid Pathway The thought that products of lipid peroxidation might include metabolites such as 5-hydroxymethyl-furanaldehyde and 2,5-furandialdehyde has attracted little, if any, attention within the biomedical research community up to this point. As will be described below, 2,5-dimethyl-furan appears to be a key intermediate in this pathway.

VI(C)1. An Overview of Lipid Peroxidation Kikugawa and Beppu (1987) have summarized present knowledge of lipid biological peroxidation, including the generation of carbonyl compounds and furans. They also noted that lipid radicals, hydroperoxides and their secondary products react with neighboring protein molecules, damaging protein structure and function. Such damage includes formation of fluorescent chromophores, lipid-protein adducts, and protein-protein crosslinks. Using SDS-polyacryl-amide gel electrophoresis, these investigators demonstrated that malonaldehyde (also known as malondialdehyde), a bifunctional molecule having two aldehyde groups, can covalently crosslink proteins. This reaction primarily involves Schiff base formation with protein ε amino groups on the sidechains of lysine residues. It is now understood that the proposed role of malondialdehyde as the primary aldehyde product of lipid peroxidation has been overstated by many investigators (Vaca, CE et al., 1988; Halliwell, B, 1984).

Gutteridge and Stocks (1976) noted that the formation of lipofuscin-type fluorescent pigments, which normally occurs slowly with aging, can be induced prematurely in experimental animals by oxygen, pro-oxidant reagents such as iron, or exposure to X-rays. The conceptual similarities between lipid peroxidation-induced protein crosslinking and protein crosslinking associated with non-enzymatic glycosylation has been noted in the research literature (Kikugawa, K and Beppu, M, 1987).

As acetaldehyde is a product of ethanol metabolism, its ability to crosslink proteins may in part underlie the etiology of alcoholic polyneuropathy, presumably by facilitating the spurious crosslinking of lysine-rich neurofilaments. Acetaldehyde has been shown to induce intermolecular crosslinking of polylysine via fluorescent complexes having an excitation maximum at 340-360 nm and an emission maximum at 410-430 nm (Kikugawa, K and Beppu, M, 1987). As large amounts of lipid are normally associated with neurofilaments (Iqbal, K et al., 1978), any physiological anomaly which might increase peroxidation in this micro-environment would predispose for neurofilament crosslinking by reactive carbonyl species.

The generation of water soluble, carbonyl-containing products of lipid peroxidation can be readily demonstrated under simple in vitro conditions (Schauenstein, E, 1967; Esterbauer, H et al., 1982). One group of recognized aldehyde-containing peroxidation products includes agents such as 4-hydroxy-2,3-trans-nonenal, which contains a reactive R-CH=CH-CHO structure (Benedetti, A et al., 1980). In vitro reaction of 4-hydroxy-2,3-trans-nonenal with phosphatidyl-ethanolamine or phosphatidylserine produces fluorescent chromolipids with an excitation maximum of 360 nm and an emission maximum of 430 nm (Esterbauer, H et al., 1986). This corresponds to the fluorescent characteristics of perox-idized microsomal lipids, which show maximal excitation at 350-360 nm and maximal emission at 430 nm. Yet much of the bio-chemistry of aldehydes generated from lipid peroxidation remains unknown. Benedetti and coworkers (1982) have noted, for example, "...it can nevertheless be concluded that 4-hydroxynonenthe research literature (Kikugawa, K and Beppu, M, 1987).

As acetaldehyde is a product of ethanol metabolism, its ability to crosslink proteins may in part underlie the etiology of alcoholic polyneuropathy, presumably by facilitating the spurious crosslinking of lysine-rich neurofilaments. Acetaldehyde has been shown to induce intermolecular crosslinking of polylysine via fluorescent complexes having an excitation maximum at 340-360 nm and an emission maximum at 410-430 nm (Kikugawa, K and Beppu, M, 1987). As large amounts of lipid are normally asso-ciated with neurofilaments (Iqbal, K et al., 1978), any physio-logical anomaly which might increase peroxidation in this micro-environment would predispose for neurofilament crosslinking by reactive carbonyl species.

The generation of water soluble, carbonyl-containing products of lipid peroxidation can be readily demonstrated under simple in vitro conditions (Schauenstein, E, 1967; Esterbauer, H et al., 1982). One group of recognized aldehyde-containing peroxidation products includes agents such as 4-hydroxy-2,3-trans-nonenal, which contains a reactive R-CH=CH-CHO structure (Benedetti, A et al., 1980). In vitro reaction of 4-hydroxy-2,3-trans-nonenal with phosphatidyl-ethanolamine or phosphatidylserine produces fluorescent chromolipids with an excitation maximum of 360 nm and an emission maximum of 430 nm (Esterbauer, H et al., 1986). This corresponds to the fluorescent characteristics of perox-idized microsomal lipids, which show maximal excitation at 350-360 nm and maximal emission at 430 nm. Yet much of the bio-chemistry of aldehydes generated from lipid peroxidation remains unknown. Benedetti and coworkers (1982) have noted, for ex-ample, "...it can nevertheless be concluded that 4-hydroxynonen-al represents only a small portion of the total amount of alde-hydes and other products derived from the peroxidative breakdown of phospholipid-bound arachidonic acid."

Some evidence has been presented which suggests that a slow, age-dependent deterioration of biological systems which counter-act lipid peroxidation may be a fundamental part of the aging process (Harman, D, 1971). This concept is sometimes referred to as the free radical theory of aging.

### VI(C)2. Generation of 2,5-Dimethyl Furan by Lipid Peroxidation

A variety of furans, aldehydes and ketones have been identified in normal human urine (Zlatkis, A and Liebich, HM, 1971; Matsumoto, KE et al., 1973). These include 2,5-dimethyl furan, 2-methyl furan, other alkyl furans, and a variety of five- to eight-carbon alkyl aldehydes and ketones.

Some of the more definitive work on the relationship between furan metabolism and lipid peroxidation has been reported by Yancey and coworkers (1986). These investigators induced lipid peroxidation in rats by use of a defined diet deficient in both vitamin E and selenium. The onset of an elevated state of in vivo lipid peroxidation was monitored by assay of red blood cell 2-thiobarbituric acid-reactive substances and assay of red blood-cell glutathione peroxidase, a selenium-dependent enzyme. TBA-reactive substances increased and glutathione peroxidase activ-ity decreased with onset of an elevated lipid peroxidation state. Capillary gas chromatographic analysis of volatile urine components identified six metabolites which were significantly increased in the vitamin E/selenium deficient animals, as shown below.

Urine samples in this study were also analyzed by trapping alde-hydes and ketones with dinitrophenylhydrazine and subsequent high performance liquid chromatography. The results showed that urine of vitamin E deficient animals contained 16 carbonyl compounds which were present at elevated levels of statistical significance. The greatest increases observed were for hydroxy-

Peak areas for those volatile metabolites with significant

| changes in concentrations due to lipid peroxodation name | | | |
|---|---|---|---|
| control | lipid | lipid peroxidation | |
| | | peroxidation | % of control |
| 2,5-dimethylfuran | 202+143 | 888+356 | 440 |
| hexanal | 1701+137 | 2784+281 | 164 |
| 2,4-pentadienal | N.D.* | 355+13 | N.D.* |
| 2-pentylfuran | 966+218 | 1843+756 | 191 |
| 2-furylmethanol | 2914+158 | 6700+901 | 230 |
| 2-decenal | 510+193 | 728+144 | 143 |

| | | | |
|---|---|---|---|
| *N.D. = not detected in control [data reproduced from Yancey, M et al., 1986, pg. 53] acetylaldehyde (676%), benzaldehyde (538%) and furfural (487%). | | | |

In discussing their findings, Yancey and coworkers concluded, in part:
Both capillary GC and LC results appear to implicate aldehydes (both normal and unsaturated) and related compounds, furan derivatives, as characteristic products of lipid peroxidation. Elevated aldehyde levels were also noticed in our earlier investigations of urinary metabolites of both long-term diabetic rats and genetically diabetic mice. Since an increased lipid peroxidation process has been associated with the diabetic condition, it is not surprising that known peroxidation metabolites should be more abundant in diabetic than normal urine samples... Increased lipid peroxidation clearly results in a greater production of metabolites that are either proven or suspected neurotoxins.

### VI(C)3. Evidence of In Vivo Oxidation of 2,5-Dimethyl-Furan

Williams (1959, pp. 550-551) also described two particular examples of in vivo furan oxidation reactions which have been demonstrated in mammals, the oxidation of 2,5-dimethyl-furan to 5-methyl-2-furoic acid and the oxidation of 5-hydroxymethylfurfural to 5-hydroxymethyl-2-furoic acid. In principle, the process of enzymatically converting hydrocarbon functional groups such as a methyl group of 2,5-dimethyl-furan to a carboxylic acid group involves three consecutive oxidation reactions. In vitro ω oxidation of heptane by rat liver microsomes, an example of a step 1 reaction, has been shown to produce several isomeric alcohols (Frommer, U et al., 1972). Step 2 in this process may be mediated by mammalian alcohol dehydrogenases (ADH), which catalyze the reversible oxidation of alcohols into aldehydes using NAD as a cofactor (McFarland, JT and Chu, Y, 1975). Kassam and coworkers (1989, pg. 569) noted earlier studies which demonstrated that mammalian liver ADH's show broad substrate specificity and reported that human class I β-1 ADH oxidizes furfuryl alcohol at approximately the same rate as ethanol.

Several mammalian aldehyde dehydrogenases have been described which have wide substrate specificities. One or more of these enzymes may be capable of catalyzing the last step in this proposed metabolic pathway, the oxidation of furanaldehydes to furancarboxylic acids. The broad specificity mammalian microsomal aldehyde dehydrogenase has been shown to oxidize aromatic substrates such as benzaldehyde as well as aliphatic substrates (Antonenkov, VD et al., 1987).

As summarized above, 2,5-dimethyl-furan is a recognized secondary product of lipid peroxidation and there is reason to believe that it may be oxidized in vivo to products such as 5-hydroxy-methyl-2-furancarboxylic acid and 2,5-furandicarboxylic acid. This, in turn, suggests that 5-hydroxymethyl furfural and 2,5-furandialdehyde may be metabolic intermediates in this process.

One report, heretofore unexplained, may represent a demonstration of the existence of the entire metabolic pathway, starting with fatty acid peroxidation and ending with recovered furancarboxylic acids. In their report on the furancarboxylic acids of human urine Mrochek and Rainey (1972) described two cases. Apart from the study presented by this inventor and coworkers on Charcot-Marie-Tooth peripheral neuropathy (Shapiro, HK et al., 1986; Shapiro, HK and Kahn, GC, 1990), these appear to be the only two examples of pathological furancarboxylic acid metabolism reported as of now in the medical literature. In their discussion of the apparent metabolic relationship of 2,5-furandicarboxylic acid, 5-hydroxymethyl-2-furoic acid, 5-hydrox-ymethyl-2-furoylglycine and 2-furoylglycine, Mrochek and Rainey (1972) described studies on two cancer patients.

Noting earlier work by Flaschentrager and coworkers, Mrochek and Rainey (1972) proposed that perhaps all four of these furan de-rivatives are products of uronic acid metabolism. Yet the ina-bility of Pettersen and Jellum (1972) to reproduce Flaschen-trager's in vivo galacturonic acid study leaves the observations of Mrochek and Rainey apparently without basis. An alternative understanding of the findings of Mrochek and Rainey is proposed herein. The exposure of living organisms to high energy radi-ation is a well recognized mechanism for initiating in vivo lipid peroxidation (Ayene, SI and Srivastava, PN, 1989; Rejhol-cova, M and Wilhelm, J, 1989: Siems, W et al., 1990). The description of the two cancer patients noted above is brief. Yet this inventor surmises that both patients may have been undergoing periodic radiation treatment. One administration of radiation therapy for patient B was noted by Mrochek and Rainey, yet presumably this was not the first such treatment. Patient A, for example, was noted to be resistant to chemotherapeutic treatment. If these patients were undergoing regularly sched-uled sessions of radiation therapy, then it is conceivable that lipid peroxidation and excretion of furancarboxylic acids may have been fluctuating according to radiation exposure. The summary of events offered by Mrochek and Rainey suggests to this inventor that they fortuitously sampled their patients at times of high lipid peroxidation and high furancarboxylic acid excre-tion, when the metabolic consequences of their most recent treatments were still in effect. When one patient was sampled again eight days after radiation exposure and found to be excreting normal levels of furancarboxylic acids, post-radiation lipid peroxidation was apparently minimal. This inventor sur-mises that the occurrence of high energy radiation exposure, a known inducer of lipid peroxidation, and supranormal excretion of furancarboxylic acids is not merely coincidental. Alternatively, one or both of the cancer patients noted above may have been receiving Adriamycin, a commonly used anti-tumor agent known for its ability to induce lipid peroxidation (Ogura, R, 1982).

It is the unique belief and understanding of this inventor that the long term generation of furanaldehyde agents as by-products of lipid peroxidation can serve as a metabolic basis or underlying contributing factor in the etiology of diabetic symptomology, the etiology of other neurological diseases featuring evidence of Schiff base type chemical crosslinking phenomena, and in the etiology of age-related symptomology.

It seems reasonable to this inventor that the hereditary motor and sensory neuropathy patients previously discussed are experiencing toxic long term consequences of furanaldehyde exposure as a consequence of defective ability to oxidize furanaldehydes which are normal products of lipid metabolism. Failure to dispose of these reactive metabolites efficiently may predispose the patients to pathological events initiated by spurious protein crosslinking.

### VI (D). Proposed Mechanism for In Vivo Trapping of 5-Hydroxymethyl-Furanaldehyde, 2,5-Furandialdehyde and Other Carbonyl-Containing Metabolites: Absorbable Pharmacological Primary Agents

For the most part, the pharmacological reactions of the present invention are based on the ability of primary amine compounds to react with aldehyde functional groups of potential-ly toxic agents, yielding covalently bound Schiff base products. Several examples of chemically analogous reactions, presented within other contexts, have been publicly presented. Representative examples are discussed below. These model chemical systems are directly analogous to the proposed mechanism of drug action which is the basis of the present invention.

In considering the specific details of the proposed drug therap-ies described herein, one of the key practical questions which arises early on is to define the normal tissue concentration of thiobartituric acid-reacting aldehydic substances in mammalian tissue. In a section of the forum discussion at the end of Dianzani's 1978 paper T. F. Slater addressed the question of endogenous levels of aldehydes. In his laboratory he observed levels of thiobarbituric acid-reactive substances in normal rat liver in the range of 0:5 umol to 1 umol/l.

A secondary beneficial aspect of the drug therapies disclosed herein may be the conservation of thiamine, or vitamin B-1. Any disease state which generates excess aldehyde metabolites may predispose for the reaction of such metabolites with the primary amino group of thiamine, effectively lowering the endogenous level of the vitamin. For similar reasons, the drug therapies disclosed herein may effect conservation of vitamin B-12 (cyanocobalamin), which has six primary amine groups (Merck Index, 11th ed, pg. 1577).

One form of application of this invention would be the prophylactic use of such procedures by healthy adult individuals in order to prevent possible onset of neurogenic diseases, and various complications thereof, such as those described above. On such a basis, this invention, or parts thereof, may be applied on an indefinite basis.

Another form of application of this invention would be its use as a medical treatment protocol for treatment of patients having diseases such as those described in sections II and III herein. As applied to a patient having a neurological disease, the intended effect of the method of treatment of this invention would be to qualitatively decrease the endogenous concentration of one or more neurotoxic aldehyde or ketone agent. This, in turn, would permit normal, slow regenerative processes to occur. As such, published studies regarding industrial exposure of humans to 2-hexanone (Allen, N et al., 1975) or acrylamide (Davenport, JG et al., 1976) provide information on patient recovery following removal from exposure to toxic agents of the kind which interfere with neurofilament metabolism, indicating that endogenous regenerative processes require between six and twelve months subsequent to removal of toxin from the environment before qualitative improvement in clinical status may be observed. Thus, by analogy, use of the method of this invention to treat patients having chronic neurodegenerative diseases may also require a minimum of six to twelve months of ongoing use before one may expect to observe improvement in clinical status.

### VI(D)1. Chemical Model Systems of Proposed Drug Action

Comments by Feeney and coworkers (1975, pg. 141) provide an appropriate introduction to this subject:
A wide variety of substances with -NH₂ groups condense with carbonyl compounds...This condensation of primary amines with aldehydes and ketones to give imines was first discovered by Schiff (1900). The overall equilibrium greatly favors hydrolysis in aqueous solution for aliphatic aldehydes. With aromatic aldehydes, the equilibrium is shifted in favor of Schiff base formation. It is important to note that increasing the nucleophilic strength of the amine will increase the rate of the carbonyl-amine reaction but will have almost no effect on the position of the equilibrium.

These comments suggest that the amine-containing carbonyl-trapping drugs described herein should have particular promise for binding furanaldehydes, which are aromatic. These comments also suggest that doses of absorbable amine drugs may require in vivo concentrations in the range of 1:100 to 1:1,000 (carbonyl: amine) in order to achieve clinical effectiveness. This, in turn, suggests that therapeutic dosages may lie in the range of grams per day and that only drugs of particularly low toxicity will have human applications. Feeney and coworkers (1975, pg. 144) also noted the phenomenon of Schiff base transimination, which occurs to a significant extent at neutral pH:

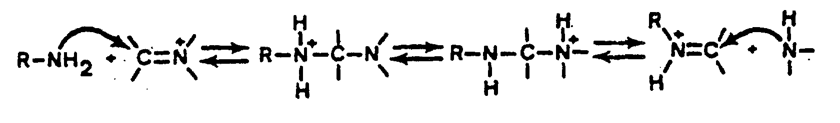

The existence of such non-enzymatic reversible transimination reactions is important within the context of this invention, as it suggests that in vivo both bound and free carbonyl agents may be sequestered by amine-containing drugs.
(a) The direct in vitro addition of p-aminobenzoic acid or ethyl p-aminobenzoate to malondialdehyde or its tautomer, β-hydroxyacrolein, has been described (Sawicki, E et al., 1963).
(b) The direct in vitro addition of n-hexylamine to β-hydroxyacrolein to produce an N,N'-disubstituted 1-amino-3-iminopropene derivative has been reported (Chio, KS and Tappel, AL, 1969). The reaction may be represented as follows: 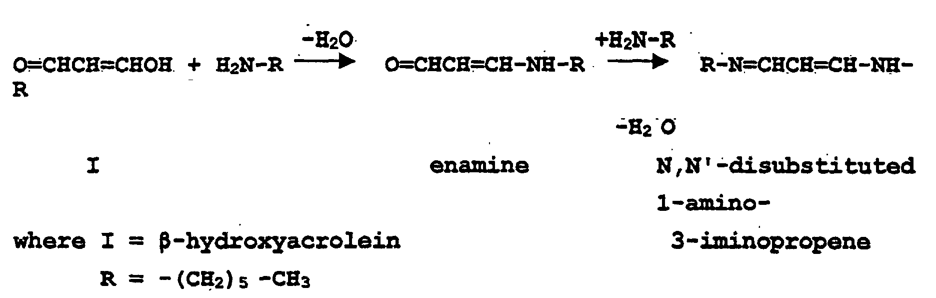
(c) The direct chemical addition of amines to 5-methyl-2-furfural has been described (Holdren, RF and Hixon, RM, 1946). A wide variety of aliphatic and aromatic primary amines can add to furfural in this manner, yielding Schiff base products (Dun-lop, AP and Peters, FN, 1953, pg. 353). 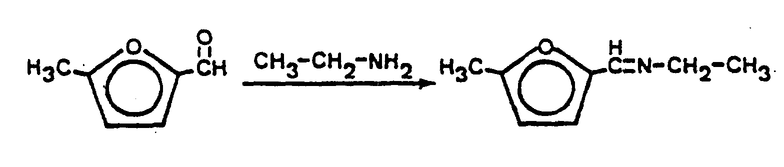 It is proposed that the small molecular weight, absorbable, primary amine drugs described herein will have analogous behavior in vivo, as well as additional characteristics which will facilitate disposal as urine metabolites. Most of these drugs, for example, contain a carboxylic acid group to facilitate uptake and processing by the kidneys.
(d) As described by Dunlop and Peters (1953, pg. 373) earlier work demonstrated the ability of furfural to react with amino-sulfonic salts to produce furfurylideneaminosulfonates: 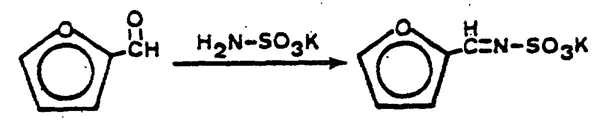
(e) The reaction of phenylaminoguanidine with furfural (Dunlop, AP and Peters, FN, 1953, pg. 371) may serve as an example of covalent furanaldehyde trapping with a hydrazine. 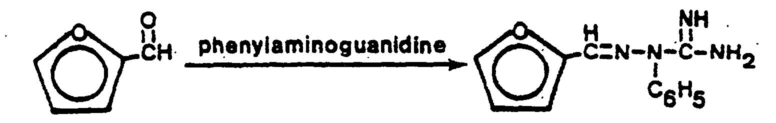
(f) Urea has been demonstrated capable of binding aldehyde compounds such as furfural (Dunlop, AP and Peters, FN, 1953, pg. 376).
(g) 5-Hydroxymethyl furfural has also been shown to directly react in vitro with two moles of urea to give a diureide (Dunlop, AP and Peters, FN, 1953, pp. 410-411).

### VI(D)2. Examples of Primary Agent Products Useful in the Present Invention

For any amino organic acid agent listed herein as useful according to the present invention, it is believed that the pharmaceutically acceptable salt forms, free acid form and ester derivatives thereof will also be useful in the claimed invention, as well as other amino organic acid chemical derivatives as specified herein.
(a) Example: p-Aminobenzoic acid (PABA) [150-13-0], including its benzene ring isomers as well as benzene ring hydroxymethyl-, methoxy-, alkyl- (1-10 carbon) substituted and hydroxyalkyl substituted derivatives. PABA is recognized as being a member of the B vitamin complex (Smith, WT, 1976, pg. 194; Winitz, M et al., 1970, pp. 527-528; Scott, CC and Robbins, EB, 1942), al-though the biochemical basis of its vitamin-like properties has not been defined. The ability of the human body to clear, i.e., excrete, metabolites of orally administered PABA is quite high (Weizman, Z et al., 1985). Recognized human urine metabolites of PABA, present in addition to the unmodified free acid, in-clude 4-acetylaminobenzoic acid, 4-aminohippuric acid and 4-acetylaminohippuric acid (Young, DS et al., 1971), as well as p-aminobenzoic acid glucuronide (Howie, MB and Bourke, E, 1979).
   Besides having vitamin-like properties and being cleared quickly by humans, PABA has also been shown to be an unusually safe drug. When screened for mutagenicity in the Ames Salmonella test, PABA was found to be non-mutagenic (Walsh, DB and Claxton, LD, 1987, pg. 62). When screened in the Ames Salmonella test in the presence of N-methyl-N'-nitro-N-nitrosoguanidine, a proven mutagen, PABA demonstrated an anti-mutagenic effect (Gichner, T et al., 1987). An analogous anti-mutagenic effect of PABA was demonstrated in experiments based on use of hairless mice exposed to ultraviolet light and a chemical carcinogen (Snyder, DS and May, M, 1975).
   Several drug products containing PABA have been marketed for human use in the United States. However, it is believed that none have been proposed as effective for the treatments claimed herein. Potassium p-aminobenzoate has been marketed as POTABA (R) in the pure form as an antifibrotic, i.e., skin softening, agent (Drug Information for the Health Care Professional, 8th ed., 1988, pgs. 111-113). As such it has been recognized for treatment of Peyronie's disease; diffuse systemic sclerosis; morphea and linear scleroderma; and dermatomyositis. For such purposes, POTABA (R) is taken orally in average doses of 12 grams/day for up to two years, although human use of 15 - 20 grams/day is recognized. As an ingredient in analgesic tablets, PABA has been marketed for domestic human use (300 mg/tablet) in PABIRIN (R) buffered tablets (with aspirin), in PABALATE (R) tablets (with sodium salicylate) and in PABALATE-SF (R) tablets (with potassium salicylate), as described in Physicians' Desk Reference, 34th ed., 1980, pgs. 849 (with aspirin) and 1430 (with salicylates). Five percent PABA in a cream base has also been marketed as a sunscreen product (Physicians' Desk Reference, 34th ed., 1980, pg. 849).
   As with the molecular basis of PABA's vitamin-like properties, the basis of its presently recognized therapeutic action has not been explicitly defined. In its summary on systemic use of PABA the Drug Information for the Health Care Professional text (8th ed., 1988, pg. 111) presented the following statement (reproduced herein its entirety):
   Mechanism of action: The mechanism by which aminobenzoate potassium exerts its antifibrotic effect is not known. It has been postulated that fibrosis results from an imbalance of serotonin and monoamine oxidase (MAO) mechanisms at the tissue level. Fibrosis is believed to occur when an excessive serotonin effect is sustained over a period of time. This could be the result of too much serotonin or too little MAO activity. Aminobenzoate potassium increases oxygen utilization at the tissue level. It has been suggested that this increased oxygen utilization could enhance the degradation of serotonin by enhancing MAO activity or other activities that decrease the tissue concentration of serotonin.

   This inventor sees no relationship of such comments to the subject matter contained herein, in particular to the use of amine drugs in the treatment of neurological diseases. Hence the clinical applications of PABA claimed in this invention are regarded by the inventor as new and novel.
   Some evidence has been publicly presented which indicates that amine agents (β-aminopropionitrile, D-penicillamine and p-aminobenzoic acid) can inhibit the transition of newly synthesized soluble collagen to highly crosslinked insoluble collagen in hamsters concomitantly treated with bleomycin to induce fibrosis (Zuckerman, JE et al., 1980). In this study on experimental pulmonary fibrosis Zuckerman and coworkers noted that
   PABA has been shown to inhibit the synthesis of glycosaminoglycans in cultured fibroblasts. Adequate tissue concentrations of glycosaminoglycans appear to be necessary for collagen deposition. The antifibrotic action of PABA may, therefore, be the result of direct inhibition of glycos-aminoglycan synthesis leading to inhibition of NSI [neutral salt insoluble] collagen accumulation.
   This proposed mechanism of action is separate from that claimed for PABA in the present invention, which is use as a covalent chemical sequestering agent for toxic carbonyl substances.
   Small molecular weight amines may act as substrates for endo-Y-glutamine:ε-lysine transferases (EC 2.3.2.13), which in theory might interfere with natural peptide crosslinking processes such as fibrin crosslinking and collagen crosslinking. However, in PABA the direct attachment of the amine group to the benzene ring is a structure which serves as a poor substrate for such enzymes (Lorand, L et al., 1979). Lorand and coworkers demonstrated that synthetic amine substrates for these transferase enzymes ideally include a sidearm structure such as H₂ N-(CH₂)₅ -X which apparently fits in a narrow active site groove. As PABA is a poor synthetic substrate for such enzymes, it apparently plays no role in normal transferase mediated protein crosslinking and its pharmacological mechanism of action has remained heretofore unexplained. What relationship, if any, the studies of Lorand and coworkers (1979) may have to that of Zuckerman and coworkers (1980) remains to be determined. The overall process of collagen crosslinking is complex, involving lysine, hydroxylysine and histidine (Tanzer, ML, 1973), and the interrelationship(s) of PABA to this process remain largely unknown.
(b) Example: p-Aminomethylbenzoic acid and analogous derivatives of the formula **H**_{**2**} **N-(CH**_{**2**}**)**_{**n**} **-C**_{**6**} **H**_{**4**} **-COOH** where n = 2-30, including m and o-benzene ring isomers of the aminoalkyl group and isomers of the aminoalkyl group where the amine is not in the omega position.
(c) Example: 4-Amino-3-methylbenzoic acid and other derivatives of PABA or benzene ring isomers thereof wherein such derivatives include from one to four additional ring substituents from the group comprising methyl group(s), ethyl group(s), or other hydrocarbon group(s) (up to 5 carbons); or substituted -OH group(s) of the structure -OCH₃, -C₂H₅ or higher molecular weight ethers (up to 5 carbons).
(d) Example: 4-Amidinobenzoic acid, **H**_{**2**} **N-C(=NH)C**_{**6**} **H**_{**4**} **-COOH.** Also included in this class are the following derivatives: 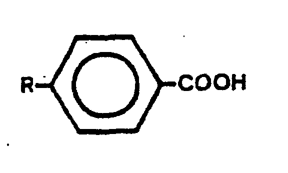 where
   - **R=**: **-NCH(=NH)NH**_{**2**}
   or **CH**_{**2**} **NCH(=NH)NH**_{**2**}
   or **(CH**_{**2**}**)**_{**n**}**NHC(=NH)NH**_{**2**}
   where n=2-10
(e) Example: p-Aminophenylacetic acid and analogous derivatives of the formula **H**_{**2**} **N-(CH**_{**2**}**)**_{**n**} **-C**_{**6**} **H**_{**4**} **-CH**_{**2**} **-COOH** where n = 1-30, as well as methyl and other sidechain hydrocarbon isomers of the aminoalkyl group, and/or hydroxylated derivatives of the side-chain aminoalkyl group, and/or derivatives bearing hydrocarbon or hydroxy substitutions at the α carbon of the acetate group.
(f) Example: 4-Amidinophenylacetic acid, **H**_{**2**}**N-C(=NH)C**_{**6**}**H**_{**4**}**-CH**_{**2**}**-COOH.**
(g) Example: 3,5-diaminobenzoic acid and other benzene ring diamine isomers.
(h) Example: 3,5-diaminoalkylbenzoic acid and benzene ring isomers, where aminoalkyl is **H**_{**2**}**N-(CH**_{**2**}**)**_{**n**}**-** and n = 1 - 30, including hydrocarbon isomers, or where aminoalkyl is **H**_{**2**}**N-(CH**_{**2**}**)**_{**m**}**-CHOH-(CH**_{**2**}**)**_{**n**}**-** where m = 0 - 15 and n = 0 - 15, including hydrocarbon isomers.
(i) Example: ρ-aminosalicylic acid. Also included in this class are the isomeric amine and hydroxy derivatives, as well as derivatives wherein the hydroxy group has been replaced by a methoxy group or alkyloxy group (2-10 carbons).
(j) Example: 4-Amino-2-sulfobenzoic acid, and structures including benzene ring isomers, derivatives where the amino group is replaced by an aminoalkyl group (1-10 carbons), and derivatives where the carboxylic acid group is replaced by a **-(CH**_{**2**}**)**_{**n**}**-COOH** group (n=1-10).

### VI (E). Prior Pharmacological Studies

Certain amine agents have recognized antioxidant properties. These include N,N'-di-(sec-butyl)-p-phenylenediamine (Scott, G, 1965, pg. 120), aniline and aniline N-subsyituted agents (Scott, G, 1965, pg. 125). In the present invention focus is placed on primary amine agents, as such agents are known to covalently react with carbonyl agents to yield Schiff base-type products (Feeney, RE et al., 1975, pg. 141). By contrast, N-substitution with hydrocarbon functional groups tends to increase amine antioxidant activity (Scott, G, 1965, pgs. 125 and 148). These are two distinct chemical phenomena. The antioxidant property of substituted amines depends on their ability to act as electron donors to alkoxy or alkylperoxy radicals (Scott, G, 1965; pgs. 127, 145 and 158). The carbonyl trapping property of primary amines depends on their ability to form Schiff base-type addition products.

Although the abilities of amines to react with alkylperoxy radicals and carbonyl groups are publicly well recognized, the application of these principles to the clinical treatment of neurological disorders and metabolically allied symptomatic phenomena is not. Within the body of information encompassing previously issued United States patents and biomedical journal publications a wide spectrum of pharmaceuticals have been described as potential therapeutic agents for one or more of the diseases falling within the context of this invention. A comprehensive review of previous public domain information conducted by this inventor has failed to reveal any examples of the claims included herein.

It should be noted that the primary agents described above in Section VI(D) have chemical structures which are fundamentally different from those of recently investigated experimental aldose reductase inhibitors. Recognized experimental aldose reductase inhibitors include sorbinil (or CP 45,634, Pfizer), tolrestat (or AY 27,773, Ayerst), statil (or ICI 128,436, I.C.I. Ltd.), ONO 2235 (ONO), M 79,175 (Eisai) and AL 1576 (Alcon). One or more primary amine functional groups are not present in the chemical structure of any of these experimental drugs (Kinoshita, JH et al., 1990, pg. 269). The structural differences between the primary agents of the present disclosure and known experimental aldose reductase inhibitors serve to underscore that these two classes of agents act by different pharmacological mechanisms. The experimental aldose reductase inhibitors are specific enzyme inhibitors. The chemical trapping agents described and claimed in the present invention should act by sequestering toxic carbonyl-containing metabolic or dietary products. Yet each of these drug classes may have therapeutic value in the treatment of secondary symptoms of diabetes.

The work encompassed by several United States patents assigned to Rockefeller University discloses the inhibition of the formation of advanced glycosylation end products of target proteins. In United States patent 4,758,583 ("Method and agents for inhibiting protein aging," Cerami, A et al., 1988) the inventors described
...an agent or compound capable of inhibiting the formation of advanced glycosylation end products of target proteins by reacting with the carbonyl moiety of the early glycosylation product of such target proteins formed by their initial glycosylation. Suitable agents may contain an active nitrogen containing group, such as a hydrazine group, and may further be at least partially derived from amino acids. Particular agents comprise aminoguanidine, alpha-hydrazinohistidine and lysine. The method comprises contacting the target protein with the composition. Both industrial and therapeutic applications for the invention are envisioned, as food spoilage and animal protein aging can be treated. ...Accordingly, the compositions useful in the present invention comprise or contain agents capable of reacting with the active carbonyl intermediate of the early glycosylation product. Suitable agents include compounds having an active nitrogen-containing group or substituent such as a hydrazine group. Also, the agent or compound may be at least partially derived from an amino acid, including the esters and amides thereof, as compounds having this derivation are generally biocompatible with the target proteins to be contacted. For example, the agent may comprise a compound selected from the group consisting of amino-guanidine, alpha-hydrazinohistidine and lysine, and possibly mixtures of these agents or compounds. Each of these agents or compounds possesses an active nitrogen-containing substituent that is believed to react with the carbonyl of the early glycosylation product. Consequently, reaction of the agents with the glycosyl-lysine of a protein would prevent this moiety from forming crosslinks with other groups.

The carbonyl-containing early glycosylation products referred to in the quotation noted above are the Amadori products of protein primary amines and various reducing sugars. No other particular compounds beyond those quoted above are claimed in US patent 4,758,583. As stated in the claims section of US patent 4,758,583, the "active nitrogen-containing" agents are hydrazine derivatives, with the exception of lysine. The claims of US patent 4,758,583 are limited to prevention of food spoilage and prevention of animal protein aging by inhibition of formation of protein advanced glycosylation end products. However, in an earlier section of US patent 4,758,583 the inventors state that
Drug therapy may be used to prevent the increased trapping and crosslinking of proteins that occurs in diabetes and aging which leads to sequelae such as arterial disease, including renal disease, hypertension, retinal damage, and extra-vascularly, damage to tendons, ligaments, and other joints. This therapy might retard atherosclerosis and connective tissue changes that occur with diabetes and aging.

It is the understanding of the present inventor that the amine compounds and their functional applications claimed herein lie beyond the claims of US patent 4,758,583. By comparison of the text of US patent 4,758,583 to the present text several substantive differences of content and inventor understanding may be noted, as summarized below.

As defined in US patent 4,758,583 the essential chemical agents are hydrazine compounds. No hydrazine compounds are claimed herein for the treatment of age- or diabetes-related etiology, although use of some hydrazine derivatives is disclosed herein regarding treatment of other diseases which feature neurodegenerative changes. The term "active nitrogen-containing group," as mentioned in US patent 4,758,583, is not defined. As such, it may conceivably refer to a diverse spectrum of thousands of nitrogen containing substances, including amino derivatives, nitro derivatives, diazonium salts, nitroso derivatives, heterocyclic bases and possibly other substances. Such a diverse spectrum of substances might include nitrofuran drugs, which are known to induce peripheral neuropathy in humans (Klinghardt, GW, 1967); trinitrotoluene, also known as TNT; or deoxyribonucleic acid, also known as DNA. The inventors of US patent 4,758,583 have not specified the meaning of the term "active nitrogen-containing group" beyond reference to hydrazine derivatives, lysine and lysine derivatives.

US patent 4,758,583 states that "...the agent or compound may be at least partially derived from an amino acid, including the esters and amides thereof...". Yet the term "amino acid" is not defined and lysine is the only example mentioned. By a narrow definition, found commonly in textbooks on chemistry and bio-chemistry, amino acids are defined as those twenty-six primary and secondary amine carboxylic acid building blocks which con-stitute the structures of peptides and proteins, which includes lysine (Morrison, RT and Boyd, RN, 1966, pp. 1098-1101). By the most broad definition, the term "amino acid" might include any chemical having some form of an amine group in its structure together with any form of organic or inorganic acid functional group, which could include carboxylic acid derivatives, phos-phoric acid derivatives, sulfonic acid derivatives, or a variety of other acidic functional groups. By this latter definition, thousands of chemical structures might be included.

The inventors of US patent 4,758,583 have not discussed or claimed application of their invention to clinical disorders featuring nerve damage beyond an inferred application to treatment of neurological deficits resulting from diabetes or aging. As such, and distinct from the present invention, they made no statement or inferred comment regarding treatment of other neurological disorders which feature cytopathological accumulations of protein and/or protein/lipid aggregates. Hence US patent 4,758,583 does not disclose the use of pharmaceuticals in treatment of Charcot-Marie-Tooth disorders, giant axon neuro-pathy, Alzheimer's pre-senile/senile dementia, Down's syndrome, Pick's disease, Parkinson's disease, amyotrophic lateral scler-osis, Huntington's disease, tinnitus, spinal muscular atrophy, Friedreich's ataxia, alcoholic polyneuropathy, multiple sclerosis, ceroid lipofuscinosis, muscular dystrophy disorders and clinically related disorders.

US patent 4,758,583 discloses that the therapeutic agents will prevent protein crosslinking associated with formation of advanced glycosylation end products by reacting with and binding to early glycosylation complexes, the Amadori products. Hence this proposed mechanism of drug action would result in drug agents indefinitely bound to the surfaces of low turnover proteins. Since such drug agents are described in US patent 4,758,583 as binding to the carbonyl group of Amadori products, the otherwise reversible relationship between Amadori product and Schiff base would no longer exist and both drug and sugar residue would remain bound to protein.

It is believed that the absorbable amine-containing drugs described herein as primary agents are weaker bases than the hydrazine derivatives of US patent 4,758,583. Hence, although most of the primary agents described herein may have some nominal ability to form addition adducts with Amadori products, this is not understood to be the primary mechanism of pharmacological action. Rather, as defined in Section VI(D)1, it is understood that the primary agents claimed herein will act most readily to combine with aldehyde groups, forming water-soluble complexes. Such primary agent-aldehyde complexes may then diffuse into the blood, if they do not initially form there, and can then be recognized by kidney tissue and sequestered into urine.

It is believed that the primary agents described herein will act primarily to bind with free aldehyde carbonyl groups such as those of unbound 5-hydroxymethyl furfural, unbound or bound 2,5-furandicarboxaldehyde, or other aldehyde products of lipid peroxidation or other sources. The binding of furanaldehydes to amino groups is recognized as being a reversible process (Keeney, M and Bassette, R, 1959). As discussed by Keeney and Bassette, all of the reactions among Amadori product, sugar Schiff base, unbound sugar, unbound furanaldehyde and bound furanaldehyde Schiff base are recognized as being reversible. This means that at any point a certain fraction of sugar in an amine-reducing sugar non-enzymatic equilibrium system is represented as unbound furanaldehyde. This process of generating non-protein bound, water soluble drug-aldehyde adducts or drug-ketone adducts may be further facilitated by the phenomenon of Schiff base transimination, as outlined in Section VI(D)1 of this present invention.

As described in US patent 4,758,583, the invention would form covalently bonded chemical addition products subsequent to reaction with reducing sugar-amine complexes, thus preventing the formation of advanced glycosylation end products. However, no explanation of the ultimate biochemical fate of such drug-sugar-amine complexes is offered, and presumably such complexes would remain attached to the surfaces of proteins. Yet success-ful treatment of the neurological sequelae of aging, diabetes and other disorders discussed in Sections II and III herein would require, in most cases, long term or indefinite drug therapy. The simple accumulation of a drug product chemically bound to proteins may be acceptable in in vitro studies and may not overtly affect the results of in vivo animal studies. Yet in order for any such proposed therapeutic agent to have safe practical application to treatment of human symptomology, some forms of effective disposal for both unreacted drug and drug-sugar-amine complexes must exist. No such mechanisms are en-visioned in US patent 4,758,583.

By contrast, the primary agents described herein include carboxylic or sulfonic acid functional groups, thus making both unreacted drugs and drug-carbonyl complexes readily recognizable by kidney tissues for active sequestration into urine. While the kidney possesses a high capacity for active removal of many aminated organic acids, the normal amino acids, such as lysine, are an exception to this rule. The kidney has no active process for removing from the blood the normal amino acids which are protein substituents. Hence, the primary agents mentioned herein have functional groups which predispose them, and presumably their trapped carbonyl derivatives, for effective, efficient removal from the body. However, the "active nitrogen-containing" substances mentioned in US patent 4,758,583 do not.

Hence the process of the present invention is not restricted to preventing early protein glycosylation complexes from transforming into advanced glycosylation end products. The present invention would effect the same end, but by a different mechanism. In the present invention water soluble complexes of drug(s) and carbonyl compounds would be formed, permitting outright removal of toxic agents from the body. As it is intended that patients be maintained on such a protocol for months, years or indefinitely, the passage of time would permit diffusion of drug-carbonyl agent complexes out of nerve and other cells and a shift in equilibria of reactions which form Amadori products, created by removal of protein-bound sugar-derived furan prod-ucts, which would have the long term effect of limiting protein crosslinking due to advanced glycosylation end products.

US patent 4,758,583 does not describe its invention as a general method of covalently trapping potentially toxic reactive alde-hyde and ketone substances which may be generated in vivo or to which a person may be exposed from an environmental source such as food. Rather, they have explicitly stated their understand-ing that each of their proposed therapeutic agents "...is be-lieved to react with the carbonyl of the early glycosylation product."

By contrast, the present inventor states herein his understanding that the primary agents claimed herein may react with a wide spectrum of aldehyde and ketone agents to form covalent adduct products. Such aldehyde and ketone agents may, for example, originate as products of lipid peroxidation, a prospect not envisioned by the inventors of US patent 4,758,583.

As the invention described herein should act to actually remove sugar by-products from the bodies of diabetics as drug-furan complexes, the present invention would serve not merely to limit protein crosslinking but would also serve to create a new mech-anism for removing excess sugar. In a simplified form, this process may be envisioned as follows:

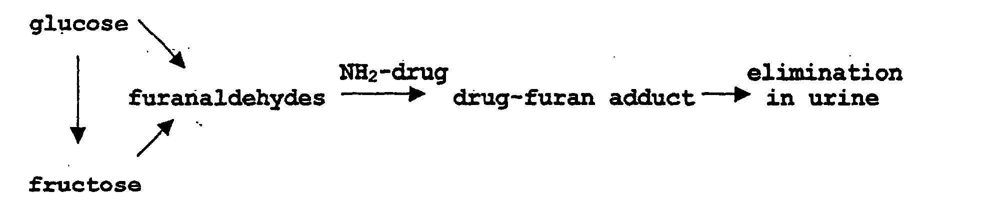

In US patent 4,900,747 ("Method and agents for removing advanced glycosylation endproducts," Vlassara, H et al., 1990) Dr. An-thony Cerami and his colleagues at Rockefeller University have described an additional invention which addresses the issues of non-enzymatic glycosylation and protein crosslinking, but which is distinct from the invention described herein. In US patent 4,900,747 the inventors described, in part, an invention wherein
...a method and associated agents are disclosed for the inhibition and treatment of protein aging in animals by stimulating the bodies of such animals to increase their recognition of and affinity for advanced glycosylation endproducts. ...The agents of the present invention comprise one or more stimulator compounds in turn, comprising a natural or synthetic advanced glycosylation endproduct alone or bound to a carrier, said carrier including a material selected from carbohydrates, proteins, synthetic poly-peptides, lipids, bio-compatible natural and synthetic resins, antigens, and mixtures thereof. The stimulator compounds could include other advanced glycosylation endproducts that may be prepared from the reaction between sugars and other macromolecules, and monokines which stimulate phagocytic cells to increase their activity toward advanced glycosylation endproducts. ...pathologies such as age related or diabetes related hardening of the arteries, skin wrinkling, arterial blockage and diabetic retinal and renal damage are all the result of the excessive build-up or trapping that occurs as the presence of advanced glycosylation endproducts increases. Accordingly, a therapeutic method in accordance with the present invention generally seeking to avert such pathologies contemplates the administration of the agents of the present invention either directly or in suitable pharmacological compositions to stimulate the phagocytic cells to remove advanced glycosylation endproducts from the body with greater speed and efficiency, and to thereby avert the onset of the pathologies recited herein. ...Thus, the present invention is predicated on the discovery that the phagocytic cells including monocytes and macrophages can be modified by exposure to certain agents or stimulator compounds that potentiate the capability of these cells with respect to their recognition and affinity for, and capability to degrade advanced glycosylation endproducts.

As summarized above, the invention embodied in US patent 4,900,747 describes use of advanced glycosylation end (AGE) products and derivatives thereof as immunostimulating agents so as to increase in vivo capacity for sequestration of AGE product-modified proteins. Hence the invention embodied in US patent 4,900,747 is based upon methodologies which are qualitatively different from those described herein. No in vitro or in vivo use of immunostimulating protocols is envisioned in the present invention.

In US patent 4,908,446 ("Inhibitors of nonenzymatic cross-linking," Ulrich, PC and Cerami, A, 1990) the inventors have elaborated on earlier work as embodied in US patent 4,758,583 to define a class of chemical derivatives of aminoguanidine such that
Accordingly, the compositions useful in the present invention comprise or contain agents capable of reacting with the active carbonyl intermediate of the early glycosylation product. Suitable agents are the hydrazine derivatives which bear an electron withdrawing group of the present invention. These agents possess an active nitrogen-containing substituent that is believed to react with the carbonyl of the early glycosylation product.

As described in US patent 4,908,446, chemical analogues of aminoguanidine were screened in vitro for their ability to inhibit glucose mediated crosslinking of bovine serum albumin. Said agents were also screened in vitro for their capacity to inhibt diamine oxidase, an unwanted effect which may conceivably limit the eventual use of such drugs for treatment of human aging and diabetes symptomology. The invention embodied in US patent 4,908,446 represents a direct extension of claims embodied in US patent 4,758,583 as regards aminoguanidine. As such, the contents of US patent 4,908,446 also lie beyond the claims of the present invention for reasons. analogous to those summarized above.

### REFERENCES CITED

Allen, N et al. "Toxic polyneuropathy due to methyl n-butyl ketone. An industrial outbreak" Arch. Neurol. 32:209-218 (1975) Antonenkov, VD et al. "On the role of microsomal aldehyde dehydrogenase in metabolism of aldehydic products of lipid peroxidation" FEBS Letters 224:357-360 (1987)
Appenzeller, O and Richardson, EP "The sympathetic chain in patients with diabetic and alcoholic polyneuropathy" Neurology (Minneap) 16:1205-1209 (1966)
Ayene, SI and Srivastava, PN "Effect of WR-2721 on radiation-induced lipid peroxidation and enzyme release in erythrocytes and microsomes" Int. J. Radiat. Biol. Relat. Stud. Phys. Chem. Med. 56:265-275 (1989)
Ballin, A et al. "Vitamin C-induced erythrocyte damage in premature infants" J. Pediatr. 113:114-120 (1988)
Baltes, W "Application of pyrolytic methods in food chemistry" J. Anal. Appl. Pyrol. 8:533-545 (1985)
Bancher, C et al. "Accumulation of abnormally phosphorylated tau precedes the formation of neurofibrillary tangles in Alzeheim-er's disease" Brain Res. 477:90-99 (1989)
Bellamy, D "Degenerative diseases of ageing as problems of natural selection. A commentary upon the concept of 'normal ageing'" Gerontology 34:315-326 (1988)
Benedetti, A et al. "Identification of 4-hydroxynonenal as a cytotoxic product originating from the peroxidation of liver microsomal lipids" Biochim. Biophys. Acta 620:281-296 (1980)
Benedetti, A et al. "Detection of carbonyl functions in phospho-lipids of liver microsomes in CCl₄- and BrCCl₃-poisoned rats" Biochim. Biophys. Acta 712:628-638 (1982)
Bhuyan, KC et al. "Lipid peroxidation in cataract of the human" Life Sci. 38:1463-1471 (1986)
Boekelheide, K "Rat testis during 2,5-hexanedione intoxication and recovery. II. Dynamics of pyrrole reactivity, tubulin content, and microtubule assembly" Toxicol. Appl. Pharmacol. 92:28-33 (1988)
Bohren, KM. et al. "Inactivation of carbonyl reductase from. human brain by phenylglyoxal and 2,3-butanedione: A comparison with aldehyde reductase and aldose reductase" Biochim. Biophys. Acta 916:185-192 (1987)
Bolton, WK and Sturgill, BC "Ultrastructure of the aging kidney" in Aging and Cell Structure, vol. 1, Johnson, Jr., JE, ed. (New York, Plenum Press, 1982) pp. 215-250
Boot-Handford, RP and Heath, H "The effect of dietary fructose and diabetes on the rat kidney" Br. J. Exp. Path. 62:398-406 (1981)
Brimijoin, S et al. "Axonal transport of dopamine-beta-hydroxyl-ase by human sural nerves in vitro" Science 180:1295-1297 (1973)
Brownlee, M "Advanced products of nonenzymatic glycosylation and the pathogenesis of diabetic complications" in Diabetes Mellitis Theory and Practice, Rifkin, H and Porte, Jr, D, eds. (New York, Elsevier, 1990) pp. 279-291
Calne, DB "Normal aging of the nervous system" in Principles of Geriatric Medicine, Andres, R, sr. ed. (New York, McGraw-Hill, 1985) pp. 231-236
Carden, MJ et al. "2,5-Hexanedione neuropathy is associated with the covalent crosslinking of neurofilament proteins" Neurochem. Pathol. 5:25-35 (1986)
Carpenter, S "Proximal axonal enlargement in motor neuron disease" Neurology 18:841-851 (1968)
Cerami, A et al. "Method and agents for inhibiting protein aging," United States Patent 4,758,583, 7/19/88
Chalmers, RA and Lawson, AM Organic Acids in Man. Analytical Chemistry, Biochemistry and Diagnosis of the Organic Acidurias (New York, Chapman and Hall, 1982)
Chio, KS and Tappel, AL "Synthesis and characterization of the fluorescent products derived from malonaldehyde and amino acids" Biochemistry 8:2821-2827 (1969)
Chojkier, M et al. "Stimulation of collagen gene expression by ascorbic acid in cultured human fibroblasts. A role for lipid peroxidation?" J. Biol. Chem. 264:16957-16962 (1989)
Chou, SM et al. "Axonal balloons in subacute motor neuron disease" J. Neuropathol. Exp. Neurol. 29:141-142 (1970)
Clements, Jr., RS "Diabetic neuropathy - New concepts of its etiology" Diabetes 28:604-611 (1979)
Cohan, SL "Neurologic diseases in the elderly" in Clinical Aspects of Aging, third edition, Reichel, W, ed. (Baltimore, Williams & Wilkens, 1989) pp. 163-176
Cornell, J et al. "Autosomal recessive inheritance of Charcot-Marie-Tooth disease associated with sensorineural deafness" Clin. Genet. 25:163-165 (1984)
Cowchock, FS et al. "X-linked motor-sensory neuropathy type-II with deafness and mental retardation: a new disorder" Am. J. Med. Genet. 20:307-315 (1985)
Creighton, MO et al. "Globular bodies: A primary cause of the opacity in senile and diabetic posterior cortical subcapsular cataracts?" Canad. J. Ophthal. 13:166-181 (1978) Davenport, JG et al. "Giant axonal neuropathy caused by industrial solvents: neurofilamentous axonal masses in man" Neurology 26:919-923 (1976)
Davidson, WS and Flynn, TG "A functional arginine residue in NADPH-dependent aldehyde reductase from pig kidney" J. Biol. Chem. 254:3724-3729 (1979)
Derrick, NM and Wishner, LA "Autoxidation of tissue lipids. I. Incorporation of dietary fatty acids and formation of monocarbonyl compounds in adipose tissue lipids of the vitamin E-deficient rat" Lipids 2:133-136 (1967)
Dianzani, MU "Biological activity of methylglyoxal and related aldehydes" Ciba Found. Symp. (67):245-270 (1978) Diplock, AT "Vitamin E, selenium and free radicals" Med. Biol. 62:78-80 (1984)
Dunlop, AP and Peters, FN The Furans (New York, Reinhold Publishing, 1953)
Dyck, PJ "Inherited neuronal degeneration and atrophy affecting peripheral motor, sensory and autonomic neurons" in Peripheral Neuropathy, 2nd ed., Dyck, PJ et al., eds. (Philadelphia, Saunders, 1984) pp. 1600-1642
Elovaara, I et al. "Immunocytochemical studies of Alzeheimer neuronal perikarya with intermediate filament antisera" J. Neurol. Sci. 62:315-326 (1983)
Engel, WK "Motor neuron histochemistry in ALS and infantile spinal muscular atrophy" in Motor Neuron Diseases: Research on Amyotrophic Lateral Sclerosis and Related Disorders, Norris, FH and Kurland, LT, eds. (New York, Grune & Stratton, 1969) pp. 218-234
Esterbauer, H et al. "Separation and characterization of the aldehydic products of lipid peroxidation stimulated by ADP-Fe2⁺ in rat liver microsomes" Biochem. J. 208:129-140 (1982) Esterbauer, H et al. "Possible involvement of the lipid-peroxid-ation product 4-hydroxynonenal in the formation of fluorescent chromolipids" Biochem. J. 239:405-409 (1986) Feeney, RE et al. "Carbonyl-amine reactions in protein chemistry" Adv. Protein Chem. 29:135-203 (1975)
Flynn, TG "Aldehyde reductases: Monomeric NADPH-dependent oxido-reductases with multifunctional potential" Biochem. Pharmacol. 31:2705-2712 (1982)
Frommer, U et al. "The monooxygenation of n-heptane by rat liver microsomes" Biochim. Biophys. Acta 280:487-494 (1972) Fujii, A et al. "Probiotics: Antistaphylococcal activity of 4-aminocyclohexanecarboxylic acid, aminobenzoic acid, and their derivatives and structure-activity relationships" J. Pharm. Sci. 66:844-848 (1977)
Fujisawa, K "An unique type of axonal alteration (so-called axonal dystrophy) as seen in goll's nucleus of 277 cases of controls. A contribution to the pathology of aging process" Acta Neuropathol. (Berl.) 8:255-275 (1967)
Gichner, T et al "Antimutagenic effect of p-aminobenzoic acid on the mutagenicity of N-methyl-N'-nitro-N-nitrosoguanidine in Salmonella typhimurium" Mutat. Res. 192:95-98 (1987)
Goebel, HH et al. "Neuropathologic and morphometric studies in hereditary motor and sensory neuropathy type II with neurofilament accumulation" Ital. J. Neurol. Sci. 7:325-332 (1986)
Gold, BG "The pathophysiology of proximal neurofilamentous giant axonal swellings: Implications for the pathogenesis of amyotro-phic lateral sclerosis" Toxicology 46:125-139 (1987)
Goodison, KL et al. "Neuronal mRNA levels are maintained in Down's brains with Alzheimer Pathology" Soc. Neurosci. Abstr. 15(pt. 2):329 (abstract 135.6) (1989)
Gottschalk, A "Interaction between reducing sugars and amino acids under neutral and acidic conditions" Glycoproteins 5B:141-157 (1972)
Graham, DG and Abou-Donia, MB "Studies of the molecular pathogenesis of hexane neuropathy. I. Evaluation of the inhibition of glyceraldehyde-3-phosphate dehydrogenase by 2,5-hexanedione" J. Toxicol. Environ. Health 6:621-631 (1980)
Graham, DG et al. "Studies of the molecular pathogenesis of hexane neuropathy. II. Evidence that pyrrole derivatization of lysyl residues leads to protein crosslinking" Toxicol. Appl. Pharmacol. 64:415-422 (1982)
Gutteridge, JM and Stocks, J "Peroxidation of cell lipids" Med. Lab. Sci. 33:281-285 (1976)
Hale, PJ et al. "Peripheral nerve concentrations of glucose, fructose, sorbitol and myoinositol in diabetic and non-diabetic patients" Diabetologia 30:464-467 (1987)
Halliwell, B "Oxygen radicals: A commonsense look at their nature and medical importance" Med. Biol. 62:71-77 (1984)
Harman, D "Free radical theory of aging: Effect of the amount and degree of unsaturation of dietary fat on mortality rate" J. Gerontol. 26:451-457 (1971)
Holdren, RF and Hixon, RM "The reaction of 2-methylfuran with formaldehyde and substituted ammonium chlorides" J. Am. Chem. Soc. 68:1198-1200 (1946)
Howie, MB and Bourke, E "Metabolism of p-aminobenzoic acid in the perfused livers of chronically uraemic rats" Clin. Sci. 56:9-14 (1979)
Hunter, MI et al. "Lipid peroxidation products and antioxidant proteins in plasma and cerebrospinal fluid from multiple sclerosis patients" Neurochem. Res. 10:1645-1652 (1985)
Hunter, MI and Mohamed, JB "Plasma antioxidants and lipid peroxidation products in Duchenne muscular dystrophy" Clin. Chim. Acta 155:123-132 (1986)
Iqbal, K et al. "Chemical relationship of the paired helical filaments of Alzeheimer's dementia to normal human neurofilaments and neurotubules" Brain Res. 142:321-332 (1978)
Jackson, MJ et al. "Techniques for studying free radical damage in muscular dystrophy" Med. Biol. 62:135-138 (1984)
Jellum, E et al. "The presence of furan derivatives in patients receiving fructose-containing solutions intravenously" Clin. Chim. Acta 47:191-201 (1973)
Johnson, PC "Diabetic neuropathy" in Current Trends in Neurosci-ences: The Pathology of the Myelinated Axon, Adachi, M, sr. ed. (New York, Igaku-Shoin, 1985) pp. 330-354
Juntunen, J et al. "Histochemically demonstrable non-specific cholinesterase as an indicator of peripheral nerve lesion in carbon disulphide-induced polyneuropathy" Acta Neuropathol. (Berl) 29:361-366 (1974)
Kaplan, RS and Wiernik, PH "Neurotoxicity of antineoplastic drugs" Semin. Oncol. 9:103-130 (1982)
Kar, NC and Pearson, CM "Catalase, superoxide dismutase, glutathione reductase and thiobarbituric acid-reactive products in normal and dystrophic human muscle" Clin. Chim. Acta 94:277-280 (1979)
Kassam, JP et al. "In vitro studies of human liver alcohol dehydrogenase variants using a variety of substrates" Drug Metab. Disp. 17:567-572 (1989)
Keeney, M and Bassette, R "Detection of intermediate compounds in the early stages of browning reaction in milk products" J. Dairy Sci. 42:945-960 (1959)
Kikugawa, K and Beppu, M "Involvement of lipid oxidation products in the formation of fluorescent and cross-linked proteins" Chem. Phys. Lipids 44:277-296 (1987)
Kinoshita, JH et al. "Aldose reductase and diabetic eye complications" in Diabetes Mellitus Theory and Practice, Rifkin, H and Porte, Jr., D, eds. (New York, Elsevier Biomedical Press, 1990) pp. 264-278
Klinghardt, GW "Schadigungen des Nervensystems durch Nitrofurane bei der Ratte" [English summary] Acta Neuropathol. (Berl.) 9:18-33 (1967)
Konecki, J et al. "Histochemical changes in the small intestine in acute furfural poisoning" Folia Histochem. Cytochem. 12:59-66 (1974)
Kousseff, BG et al. "Charcot-Marie-Tooth disease with sensorineural hearing loss-autosomal dominant trait" Birth Defects 18:223-228 (1982)
Krasavage, WJ et al. "The relative neurotoxicity of methyl-n-butyl ketone, n-hexane and their metabolites" Toxicol. Appl. Pharmacol. 52:433-441 (1980)
Lamarche, J et al. "Ultrastructural observations on spinal ganglion biopsy in Friedreich's ataxia: A preliminary report" Can. J. Neural. Sci. 9:137-139 (1982)
Lampert, P et al. "An electron microscopic study of dystrophic axons in the gracile and cuneate nuclei of vitamin E-deficient rats" J. Neuropath. Exp. Neurol. 23:60-77 (1964)
Lawson, AM et al. "Urinary organic acids in man. I. Normal patterns" Clin. Chem. 22:1283-1287 (1976)
Lee, S et al. "A study of infantile motor neuron disease with neurofilament and ubiquitin immunocytochemistry" Neuropediatrics 20:107-111 (1989)
Lever, M et al. "Automated fluorimetric determination of furfur-als" Anal. Biochem. 144:6-14 (1985)
Lorand, L et al. "Specificity of guinea pig liver transglutaminase for amine substrates" Biochemistry 18:1756-1765 (1979)
Marsden, CD "Neuromelanin and Parkinson's disease" J. Neural Transm. Suppl. 19:121-141 (1983)
Matsumoto, KE et al. "The identification of volatile compounds in human urine" J. Chromatogr. 85:31-34 (1973)
Mattson, J and Mattson, MP "Relationship of neurodegeneration to the expression of Alzeheimer-associated antigens in cultured hippocampal neurons" Soc. Neurosci. Abstr. 15(pt. 2):1039 (1989)
McFarland, JT and Chu, Y "Effect of pH on the liver alcohol dehydrogenase reaction" Biochemistry 14:1140-1146 (1975)
Mendell, JR et al. "Toxic polyneuropathy produced by methyl n-butyl ketone" Science 185:787-789 (1974)
Mevissen, L and Baltes, W ["Model experiments for the Maillard reaction. VII. Identification of volatile compounds from the reaction of D-glucose with L-phenylalanine in an aqueous medium"] Z. Lebensm. Unters. Forsch. 176:206-207 (1983)
Miller, CC et al. "Alzeheimer's paired helical filaments share epitopes with neurofilament side arms" EMBO J. 5:269-276 (1986) Miyagishi, T et al. "Electron microscopic studies on the lipo-pigments in the cerebral cortex nerve cells of senile and vitamin E deficient rats" Acta Neuropath. 9:7-17 (1967)
Moran, MA and Gomez-Ramos, P "Initial stages of tangle formation in degenerating neurons of aged and Alzeheimer patients" Soc. Neurosci. Abstr. 15(pt. 2):1039 (abstract 414.8) (1989)
Morrison, RT and Boyd, RN Organic Chemistry, 2nd ed. (Boston, Allyn and Bacon, 1966)
Mrochek, JE and Rainey, Jr, WT "Identification and biochemical significance of substituted furans in human urine" Clin. Chem. 18:821-828 (1972)
Murty, BS et al. "Levels of 5-hydroxymethylfurfural in dextrose injection" Am. J. Hosp. Pharm. 34:205-206 (1977)
Ochoa, J and Mair, WG "The normal sural nerve in man. II. Changes in the axons and Schwann cells due to ageing" Acta Neuropath. (Berl.) 13:217-239 (1969)
Ogura, R "Adriamycin-induced lipid peroxidation and its protection" in Lipid Peroxides in Biology and Medicine, Yagi, K, ed. (New York, Academic Press, 1982) pp. 255-270
Oppenheimer, DR "Diseases of the basal ganglia, cerebellum and motor neurons" in Greenfield's Neuropathology, Blackwood, W and Corsellis, JAN, eds. (Chicago, Year Book Medical Publishers, 1976) pp. 608-651
Osuntokun, BO "Toxic and nutritional peripheral neuropathies" in International Conference on Peripheral Neuropathies (Princeton, Excerpta Medica, 1982) pp. 130-144
Palmer, MH Urinary Incontinence (Thorofare, NJ, Slack Publishing, 1985)
Parhad, IM et al. "Doxorubicin intoxication: Neurofilamentous axonal changes with subacute neuronal death" J. Neuropathol. Exp. Neurol. 43:188-200 (1984)
Perry, G et al. "Paired helical filaments from Alzeheimer disease patients contain cytoskeleton components" Proc. Nat. Acad. Sci. (USA) 82:3916-3920 (1985)
Pettersen, JE and Jellum, E "The identification and metabolic origin of 2-furoylglycine and 2,5-furandicarboxylic acid in human urine" Clin. Chim. Acta 41:199-207 (1972)
Pinkston, D et al. "High-resolution gas chromatography-mass spectrometry of the methyl esters of organic acids from uremic hemofilrates" J. Chromatogr. 223:1-19 (1981)
Pipeleers, DG et al. "Microtubule assembly and the intracellular transport of secretory granules in pancreatic islets" Science 191:88-90 (1976)
Pongor, S et al. "Aging of proteins: Isolation and identification of a fluorescent chromophore from the reaction of poly-peptides with glucose" Proc. Natl. Acad. Sci. (USA) 81:2684-2688 (1984)
Powell, HC et al. "Schwann cell abnormalities in 2,5-hexanedione neuropathy" J. Neurocytol. 7:517-528 (1978)
Prineas, JW "The pathogenesis of dying-back polyneuropathies. Part I. An ultrastructural study of experimental tri-orthocresyl phosphate intoxication in the cat" J. Neuropath. Exp. Neurol. 28:571-597 (1969)
Prineas, JW et al. "Giant axonal neuropathy - A generalized disorder of cytoplasmic microfilament formation" J. Neuropathol. Exp. Neurol. 35:458-470 (1976)
Rao, GN and Cotlier, E "Free epsilon amino groups and 5-hydroxymethylfurfural contents in clear and cataractous human lenses" Invest. Ophthalmol. Vis. Sci. 27:98-102 (1986)
Rejholcova, M and Wilhelm, J "Time course of lipolytic activity and lipid peroxidation after whole-body gamma irradiation of rats" Radiat. Res. 117:21-25 (1989)
Rice, EW "Furfural: exogenous precursor of certain urinary furans and possible toxicologic agent in humans" Clin. Chem. 18:1550-155.1 (1972)
Rosenbaum, E et al. "Nephropathy in sucrose-fed rats. Electron and light microscopic studies" Diabetes 20:803-810 (1971)
Sawicki, E et al. "Comparison of spectrophotometric and spectro-photofluorometric methods for the determination of malonalde-hyde" Anal. Chem. 35:199-205 (1963)
Scallet, BL and Gardner, JH "Formation of 5-hydroxymethylfurfural from D-glucose in aqueous solution" J. Am. Chem. Soc. 67:1934-1935 (1945)
Schauenstein, E "Autoxidation of polyunsaturated esters in water: Chemical structure and biological activity of the products" J. Lipid Res. 8:417-428 (1967)
Schmucker, DL "Subcellular and molecular mechanisms underlying the age-related decline in liver drug metabolism" in The Aging Process: Therapeutic Implications, Butler, RN and Bearn, AG, eds. (New York, Raven Press, 1985) pp. 117-134
Schwendemann, G "Diagnosis of juvenile ceroid-lipofuscinosis by electron microscopy of lymphocytes and of rectal, skin and sural nerve biopsy tissues" in Ceroid-Lipofuscinosis (Batten Disease), Armstrong, D, sr. ed. (New York, Elsevier Biomedical Press, 1982). pp. 117-136
Scott, CC and Robbins, EB "Toxicity of p-aminobenzoic acid" Proc. Soc. Exp. Biol. Med. 49:184-186 (1942)
Scott, G "Antioxidants: Radical chain-breaking mechanisms" in Atmospheric Oxidation and Antioxidants (New York, Elsevier Publishing Co., 1965) pp. 115-169
Seifter, S and Englard, S "Carbohydrate metabolism" in Diabetes Mellitus Theory and Practice, Rifkin, H and Porte, Jr., D, eds. (New York, Elsevier Biomedical Press, 1990) pp. 1-40
Selkoe, DJ et al. "Alzeheimer's disease: insolubility of partially purified paired helical filaments in sodium dodecyl sulfate and urea" Science 215:1243-1245 (1982)
Shankar, SK et al. "Immunocytochemical characterization of neurofibrillary tangles in amyotrophic lateral sclerosis and Parkinsonism-dementia of Guam" Ann. Neurol. 25:146-151 (1989)
Shapiro, HK et al. "Metabolic screening of Charcot-Marie-Tooth disease patients by gas chromatography/mass spectrometry" Muscle & Nerve 9(suppl. 5S):128 (1986)
Shapiro, HK and Kahn, GC "Metabolic screening studies on Charcot-Marie-Tooth disease" in Charcot-Marie-Tooth Disorders: Pathophysiology, Molecular Genetics, and Therapy, Lovelace, RE and Shapiro, HK, eds. (New York, Wiley-Liss, 1990) pp. 365-371
Shelanski, ML and Wisniewski, H "Neurofibrillary degeneration induced by vincristine therapy" Arch. Neurol. 20:199-206 (1969)
Shimasaki, H et al. "Formation of age pigment-like fluorescent substances during peroxidation of lipids in model membranes" Biochim. Biophys. Acta 792:123-129 (1984)
Shimizu, J and Watanabe, M "Gas chromatographic analysis of furfural and hydroxymethyl-furfural in wine" Agric. Biol. Chem. 43:1365-1366 (1979)
Sidenius, P and Jakobsen, J "Reversibility and preventability of the decrease in slow axonal transport velocity in experimental diabetes" Diabetes 31:689-693 (1982)
Siems, W et al. "Long term effects of monthly low dose whole body irradiation on the glutathione status and thiobarbituric acid-reactive substances in different organs of male Wistar rats" Radiobiol. Radiother. (Berl) 31:257-263 (1990)
Sima, AF "Structural and functionbal characterization of the neuropathy in the spontaneously diabetic BB-Wistar rat" in Diabetic Neuropathy, Gato, Y, sr. ed. (Princeton, Excerpta Medica, 1982)
Slight, SH et al. "Glycation of lens proteins by the oxidation products of ascorbic acid" Biochim. Biophys. Acta 1038:367-374 (1990)
Smith, WT "Nutritional deficiencies and disorders" in Greenfield's Neurology, Blackwood, W and Corsellis, JAN, eds. (Chicago, Year Book Medical Publishers, 1976) pp. 194-237
Snyder, DS and May, M "Ability of PABA to protect mammalian skin from ultraviolet light-induced skin tumors and actinic damage" J. Invest. Dermatol. 65:543-546 (1975)
Spencer, PS et al. "The enlarging view of hexacarbon neurotoxicity" CRC Crit. Rev. Toxicol. 7:279-356 (1980)
Steinbrecher, UP "Oxidation of human low density lipoprotein results in derivatization of lysine residues of apolipoprotein B by lipid peroxide decomposition products" J. Biol. Chem. 262:3603-3608 (1987)
Stuckey, BN "Antioxidants as food stabilizers" in CRC Handbook of Food Additives, Furia, TE, ed. (West Palm Beach, FL, CRC Press, 1968) pp. 209-245
Sumpter, PQ et al. "An ultrastructural analysis of the effects of accumulation of neurofibrillary tangle in pyrimidal neurons of the cerebral cortex in Alzeheimer's disease" Neuropathol. Appl. Neurobiol. 12:305-319 (1986)
Tan, NT et al. "Neuropathology of the cortical lesions of the Parkinsonian-dementia (PD) complex of Guam" Clin. Exp. Neurol. 17:227-234 (1981)
Tanzer, ML "Cross-linking of collagen. Endogenous aldehydes in collagen react in several ways to form a variety of unique covalent cross-links" Science 180:561-566 (1973)
Tappel, AL "Biological antioxidant protection against lipid per-oxidation damage" Am. J. Clin. Nutr. 23:1137-1139 (1970)
Tellez-Nagel, I et al. "Studies on brain biopsies of patients with Huntington's chorea" J. Neuropathol. Exp. Neurol. 33:308-332 (1974)
Tiller-Borcich, JK and Forno, LS "Parkinson's disease and dementia with neuronal inclusions in the cerebral cortex: Lewy bodies or Pick bodies" J. Neuropathol. Exp. Neurol. 47:526-535 (1988)
Tomlinson, DR and Mayer, JH "Defects of axonal transport in diabetes mellitus - A possible contribution to the aetiology of diabetic neuropathy" J. Auton. Pharmac. 4:59-72 (1984) Tsuchida, M et al. "Lipofuscin and lipofuscin-like substances" Chem. Phys. Lipids 44:297-325 (1987)
Ulbricht, RJ et al. "A review of 5-hydroxymethylfurfural (HMF) in parenteral solutions" Exp. Appl. Toxicol. 4:843-853 (1984) Ulrich, PC and Cerami, A "Inhibitors of nonenzymatic cross-linking," United States Patent 4,908,446, 3/13/90
Vaca, CE et al. "Interaction of lipid peroxidation products with DNA. A review" Mut. Res. 195:137-149 (1988) Varma, SD et al. "Diabetic cataracts and flavonoids" Science 195:205-206 (1977)
Vlassara, H et al. "Method and agents for removing advanced glycosylation end products," United States Patent 4,900,747, 2/13/90
Walsh, DB and Claxton, LD "Computer-assisted structure-activity relationships of nitrogenous cyclic compounds tested in Salmonella assays for mutagenicity" Mutat. Res. 182:55-64 (1987)
Ward, JD et al. "Effect of blood sugar control on the accumulation of sorbitol and fructose in nervous tissues" Diabetes 21:1173-1178 (1972)
Weizman, Z et al. "Bentiromide test for assessing pancreatic dysfunction using analysis of para-aminobenzoic acid in plasma and urine" Gastroenterology 89:596-604 (1985)
Williams, RT Detoxication Mechanisms: The Metabolism and Detoxi-cation of Drugs, Toxic Substances and Other Organic Compounds (New York, John Wiley & Sons, 1959)
Winitz, M et al. "Studies in metabolic nutrition employing chem-ically defined diets. I. Extended feeding of normal human adult males" Am. J. Clin. Nutr. 23:525-545 (1970)
Wisniewski, HM et al. "Neurofibrillary pathology" J. Neuropath. Exp. Neurol. 29:163-176 (1970)
Wisniewski, HM et al. "Neurofibrillary and synaptic pathology in the aged brain" in Aging and Cell Structure, volume 1, Johnson, Jr., JE, ed. (New York, Plenum Press, 1982) pp. 105-142
Yamamura, Y et al. "Morphological studies on human and experimental diabetic neuropathy" in Diabetic Neuropathy, Goto, Y, sr. ed. (Princeton, Excerpta Medica, 1982) pp. 80-85
Yancey, M et al. "Quantitative alterations in the metabolism of carbonyl compounds due to diet-induced lipid peroxidation in rats" J. Chromatogr. 382:47-56 (1986) Ylikoski, J et al. "Vestibular nerve in Meniere's disease" Arch. Otolaryngol. 106:477-483 (1980)
Yoshimura, N "Topography of Pick body distribution in Pick's disease: a contribution to understanding the relationship between Pick's and Alzeheimer's diseases" Clin. Neuropath. 8:1-6 (1989)
Young, DS et al. "Influence of a chemically defined diet on the composition of serum and urine" Clin. Chem. 17:765-773 (1971)
Zlatkis, A and Liebich, HM "Profile of volatile metabolites in human urine" Clin. Chem. 17:592-594 (1971)
Zuckerman, JE et al. "Evaluation of antifibrotic drugs in bleomycin-induced pulmonary fibrosis in hamsters" J. Pharmacol. Exp. Ther. 213:425-431 (1980)

## Claims

1. Use of a primary agent; said primary agent being a free acid, pharmaceutically acceptable salt, benzene ring isomer, carboxylic acid ester derivative or sulfonic acid ester derivative of a water soluble, low molecular weight substance (100 to 1,100 range of molecular weights) containing a primary amine chemical functional group selected from the group consisting of:
a. p-aminobenzoic acid (PABA);
b. p-aminomethylbenzoic acid and analogous derivatives of the formula H₂ N - (CH₂)ₙ - C₆H₄ - COOH where n = 2-30, including m- and o-benzene ring isomers of the aminoalkyl group and isomers of the aminoalkyl group where the amine is not in the ω position;
c. 4 Amino-3-methylbenzoci acid and other derivatives of PABA or benzene ring isomers thereof wherein such derivatives indude from one to four additional ring substituents from the group consisting of methyl, ethyl, or other hydrocarbon (up to 5 carbons); substituted -OH of the structure -OCH₃,- C₂H₅ or higher molecular weight ethers (up to 5 carbons);
d. 4-amidinobenzoic acid, H₂N-C(=NH)C₆H₄-COOH, and the following derivatives thereof: 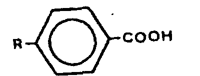 where R= -NHC(=NH)NH₂, CH₂ NHC(=NH)NH₂ or (CH₂)ₙ NHC(= NH) NH₂ where n = 2 - 10;
e. p-aminophenylacetic acid and analogous derivatives of the formula H₂ N-(CH₂ )ₙ - C₆H₄ - CH₂ -COOH where n = 1-30, as well as methyl and other sidechain hydrocarbon isomers of the aminoalkyl group, and/or hydroxylated derivatives of the sidechain aminoalkyl group, and/or derivatives bearing hydrocarbon or hydroxy substitutions at the a carbon of the acetate group;
f. 4-amidinophenylacetic acid, H₂ N-C(=NH)C₆ H₄ - CH₂ - COOH;
g. 3,5 diaminobenzoic acid and other benzene ring diamine isomers;
h. 3,5 diaminoalkylbenzoic acid and other benzene ring isomers, where aminoalkyl is H₂ N-(CH₂)ₙ₋ and n = 1 - 30, including hydrocarbon isomers, or where aminoalkyl is H₂ N-(CH₂)ₘ-CHOH - (CH₂)ₙ₋ where m = 0 - 15 and n = 0 -15 including hydrocarbon isomers thereof;
i. p-aminosalicylic acid, and the isomeric amine and hydroxy derivatives thereof, as well as derivatives wherein the hydroxy has been replaced by methoxy or alkyloxy having 2 - 10 carbons;
j. 4-amino-2-sulfobenzoic acid, and derivatives thereof including benzene ring isomers and derivatives where the amino group is replaced by an aminoalkyl group having 1-10 carbons, and derivatives where the carboxylic acid group is replaced by a
-(CH₂)ₙ-COOH group (n = 1 - 10);
for preparing a pharmaceutical composition intended for oral administration for treating the symptoms of hereditary motor and sensory neuropathies; giant axonal neuropathy; diabetic polyneuropathy or metabolic symtomology related thereto; Alzheimer's presenile dementia; Alzheimer's senile dementia; Down's syndrome; Pick's disease; Parkinson's disease; amyotrophic lateral sclerosis; disorders clinically related to the aforementioned neurological diseases; Huntington's disease; tinnitus; spinal muscular atrophy; Friedreich's ataxia; alcoholic polyneuropathy; multiple sclerosis; ceroid lipofuscinosis; age-related atrophy of peripheral sensory and motor nerves; age-related atrophy of autonomic nerves including symptoms of hypoperistalsis of the alimentary tract, hiatal hernia (partial food regurgitation), urinary incontinence, breathing insufficiency due to diaphragm weakness and decreased autonomic sexual function; age-related atrophy of neurons of the central nervous system; muscular dystrophy disorders; age-onset pathophysiologically related changes in the cardiovascular system, kidney and optic lens; or atherosclerosis; in combination with at least one required co-agent selected from the closed group of antioxidants consisting of vitamin E (a tocopherol), selenium, citric acid, ubiquinol, a seleno-containing amino acid, glutathione and sulfhydryl containing proteins, the closed group of vitamins consisting of vitamin A, vitamin D, vitamin K and vitamin B-6, the closed group of hormone consisting of human growth hormone, the closed group of chemical conjugating substance which facilitates kidney drug elimination consisting of glycine, the closed group of metabolite at risk of depletion consisting of pantothenic acid, the closed group of sulfhydryl containing substances consisting of cysteine, homocysteine, methionine and thioctic acid (a lipoic acid); and optionally in combination with a co-agent selected from the group consisting of suspending reagents or the functional equivalent thereto.

2. Use according to claim 1, wherein said pharmaceutical is in dosage unit form for administration of said primary agent in a dosage in the range of 600 mg/day to 40 grams/day.

3. Use according to claims 1 or 2, wherein said primary agent inhibits the reaction between (1) the disease-induced or disease-related carbonyl-containing aliphatic or aromatic hydrocarbons and (2) intracellular structures which, in the absence of said primary agent, yields covalently bonded crosslinked products.

4. Use according to claim 3, wherein the covalent bond crosslinking additionally comprises at least one neuropathological structure selected from the group consisting of:
a. polymerized aggregates of structural protein filaments such as excess neurofilament accumulation;
b. heterogeneous protein aggregates such as neurofibrillary tangles;
c. amorphous protein and lipid aggregates, such as senile plaques; or
d. lipfuscin granules.

5. Use according to claim 3 or claim 4, wherein the primary agent is a water soluble, small molecular weight chemical having at least one primary amine group thereon for reaction with carbonyl groups to yield covalently bonded Schiff base products, and wherein the primary agent is selected from the group as defined in claim 1.

6. Use according to claim 3, wherein the primary agent is additionally **characterized in that** it does not interact with the normal cell metabolism of said human or does so in a non-cytotoxic manner, is capable of being tolerated by said human in dosages in the range of 600 mg/day to 40 grams/day for extended periods of time and wherein the primary agent is readily absorbed by the kidney tissue and excreted in the urine without nephrotoxic consequences.

7. A pharmaceutical composition intended for oral administration for use in the treatment of symptoms of hereditary motor and sensory neuropaties; giant axonal neuropathy; diabetic polyneuropathy or metabolic symtomology related thereto; Alzheimer's presenile dementia; Alzheimer's senile dementia; Down's syndrome; Pick's disease; Parkinson's disease; amyotrophic lateral sclerosis; disorders clinically related to the aforementioned neurological diseases; Huntington's disease; tinnitus; spinal muscular atrophy; Friedreich's ataxia; alcoholic polyneuropathy; multiple sclerosis; ceroid lipofuscinosis; age-related atrophy of peripheral sensory and motor nerves; age-related atrophy of autonomic nerves including symptoms of hypoperistalsis of the alimentary tract, hiatal hernia (partial food regurgitation), urinary incontinence, breathing insufficiency due to diaphragm weakness and decreased autonomic sexual function; age-related atrophy of neurons of the central nervous system; muscular dystrophy disorders; age-onset pathophysiologically related changes in the cardiovascular system, kidney and optic lens; or atherosclerosis; comprising an effective amount of at least one primary agent of claim 1 in combination with a required co-agent selected from the group of antioxidants, vitamins, a hormone, the chemical conjugating substance which facilitates kidney drug elimination, the metabolite at risk of depletion, and sulfhydryl containing substances of claim 1 optionally in association with a pharmaceutically acceptable carrier thereof.

## Patentansprüche

1. Verwendung eines pimären Wirkstoffs; besagter pimärer Wirkstoff ist eine freie Säure, pharmazeutisch akzeptables Salz, Benzolringisomer, Carboxylsäureester Derivat oder Sulfonsäureester Derivat einer wasserlöslichen Substanz niedrigem Molekulargewichts ( zwischen 100 und 1100 Molekulargewicht), welches als funktionale Gruppe ein pimäres Amin aus der nachfolgend aufgeführten Guppe enthält:
a. p-Aminobenzoesäure (PABA)
b. p-Aminomethylbenzoesäure and analoge Derivate gemäß der Formel H₂N - (CH₂)ₙ - C₆K₄ - COOH , wobei n = 2 - 30, einschließlich m- und o-Benzolring-isomere der Aminoalkylgruppe oder Isomere der Aminoalkylgruppe, in denen das Amin nicht in der ω-Position ist.
c. 4 Amino-3-methylbenzoesäure und andere PABA-Derivate oder Benzolringisömere davon, einschließlich der Derivate die zwischen einem bis zu vier zusätzlichen Ring Substituenten aus der Gruppe Methanol, Äthanol oder anderer Kohlenwasserstoffe (bis zu 5 Kohlenstoff Atome) enthalten; Ersatz von -OH der. Strukturen -OCH₃, -C₂H₅ oder Ätheren mit höherem Molekulargewicht (bis zu 5 Kohlenstoffatome);
d. 4-Amidinobenzoesäure, H₂N-C(=NH)C₆H₄-COOH und ihre nachfolgend benannten Derivate: 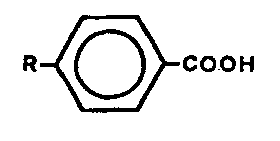 wobei R = -NHC(=NH)NH₂, CH₂ NHC(=NH)NH₂ oder (CH₂)ₙ NHC(=NH)NH₂ und miti n = 2 - 10;
e. p-Aminophenylessigsäure und analoge Derivate der Formel H₂N - (CH₂)ₙ-C₆H₄ - CH₂ - COOH, wobei n = 1-30, als auch Methanol und andere Seitenkette Kohlenwasserstoff Isomere der Aminoalkyl Gruppe, und/oder hydroxylierte Derivate der Aminoalkylgruppe Seitenkette, und/oder Derivate die Kohlenwasserstoff oder Hydroxy-Substitutionen am α-Kohlenstoffatom der Azetat Gruppe tragen;
f. 4-Amidinophenylessigsäure, H₂N-C(=NH)C₆H₄ - CH₂ - COOH;
g. 3,5 Diaminobenzoesäure und andere Benzolring Diaminisomere;
h. 3,5 Diaminoalkylbenzoesäure und andere Benzolring Isomere, wobei das Aminoalkyl gleich H₂N-(CH2)ₙ ist, mit n = 1 - 30, einschließlich Kohlenwasserstoff Isomere, oder wobei das Aminoalkyl gleich H₂N-(CH₂)ₘCHOH- (CH₂)ₙ ist, wobei m = 0 -15 und n = 0 -15, einschließlich der Kohlenwasserstoff Isomere davon;
i. p-Aminosalizylsäure, und die isomeren Amin- und Hydroxy-Derivate davon, als auch Derivate in denen das Hydroxy mit einem Methoxy oder Alkyloxy mit 2 - 10 Kohlenstoffatomen ersetzt wurde.
j. 4-Amino-2-Sulfobenzolsäure, und ihre Derivate einschließlich Benzolring Isomere und Derivate, in denen die Aminogruppe mit einer Aminoalkylgruppe mit 1-10 Kohlenstoffatomen ersetzt ist, und Derivate in denen die Carboxylsäure mit der Gruppe -(CH₂)ₙ - COOH ersetzt wurde (n =1-10);
zur Herstellung eines pharmazeutischen Präparats für orale Einnahme zur Verwendung in der Behandlung der Symptome erblicher Neuropathien des Bewegungs- und Wahrnehmungssystems, der Riesen-Axonpathie, diabetischer Polyneuropathie oder dazu verwandter metabolischer Symptomatik, präseniler Alzheimer Demenz, seniler Alzheimer Demenz, Down's Syndrom, Pick's Krankheit, Parkinson Krankheit, amyotrophischer lateraler Sklerose, von Beschwerden die klinisch mit den obenerwähnten neurologischen Krankheiten verbunden sind, Huntington's Chorea, Ohrensausen, spinaler Muskelatrophie, Friedreich Ataxie, Alkohol-bedingter Polyneuropathie, multipler Sklerose, Ceroidlipofuscinose, altersbedingter Atrophie des periphären Nervensystems des Wahrnehmungs- und Bewegungstrakts, alterbedingter Atrophie des autonomen Nervensystems, einschließlich der Symptome der Hypoperistaltik des Verdauungstrakts, der Zwerchfellhernie (partieller Nahrungsrückstau), Harninkontinenz, von Atmungsbeschwerden aufgrund von Zwerchfellschwäche und verminderter autonomer Sexualfunktionen, altersbedingter Atrophie der Nervenzellen des zentralen Nervensystems, Muskeldistrophie, altersbedingter pathophysiologisch bedingter Veränderungen des Kreislaufs, der Nieren und der Augenlinse, Arteriosklerose,
in Verbindung mit mindestens einem erforderlichem Zusatzmittel aus der beschränkten Gruppe der Anitoxidans, welche aus Vitamin E (α tocopherol), Selen, Zitrussäure, Ubiquinol, einer selenhaltigen Aminosäure, Glutathione und sulfohyrylhaltigen Proteinen besteht; die beschränkte Gruppe von Vitaminen, die aus Vitamin A, Vitamin D, Vitamin K und Vitamin B-6 besteht; die beschränkte Gruppe von Hormonen die aus menschlichem Wachstumshormon besteht; die beschränkte Gruppe von chemisch conjugierten Substanzen welche die Drogenausscheidung in den Nieren erleichtern, welche aus Glycin besteht; die beschränkte Gruppe von Metaboliten mit Risiko der Depletion bestehend aus Pantothensäure; die beschränkte Gruppe der sulfohydrylhaltigen Substanzen bestehend aus Cystein, Homocystein, Methionin und Thioktansäure (α Liponsäure); und wahlweise in Verbindung mit einem Zusatzmittel welches aus der Gruppe der Suspensionsmittel oder funktional äquivalenten Mittel entstammt.

2. Verwendung gemäß des Anspruchs Nr. 1, wobei das besagte Pharmazeutikum in einer dosierten Form zur Verabreichung des besagten Wirkstoffs in einer Dosierung im Bereich von 600 mg/Tag bis zu 40 g/Tag ist.

3. Verwendung gemäß des Anspruchs Nr. 1 und Nr. 2, worin besagter primärer Wirkstoff die Reaktion zwischen (1) den krankheitsbedingten oder krankheitsverbundenen carbonylhaltigen aliphatischen oder aromatischen Kohlenwasserstoffen und den (2) intrazellulären Strukturen unterbindet, welche, in der Abwesenheit des besagten primären Wirkstoffs, kovalent gebundene, vernetzte Produkte bilden.

4. Verwendung gemäß Anspruch Nr. 3, worin die kovalent verbundenen Netzwerke darüber hinaus mindestens eine der nachfolgend aufgeführten Gruppe neuropathologischer Strukturen bildet:
a. polimerisierte Aggregate struktureller Proteinfilamenten wie z.B. überschüßige Ablagerungen von Neurofilamenten;
b. heterogene Proteinaggregate wie z.B. neurofibilläre Bündel;
c. amorphe Protein und Lipid Aggregate, wie z.B. senile Plaques, oder
d. Lipfuscin Körperchen

5. Verwendung gemäß Anspruch Nr. 3 oder 4, worin der primäre Wirkstoff eine wasserlösliche chemische Substanz niedrigem Molekulargewichts ist, welche mindestens eine primären Amingruppe zur Reaktion mit Carbonylgruppen enthält, um kovalent gebundene Schiff's Basenprodukte zu bilden, und worin der primäre Wirkstoff von der Gruppe, die in Anspruch Nr. 1 definiert wurde, ausgewählt ist.

6. Verwendung gemäß Anspruch Nr. 3, worin der primäre Wirkstoff zusätzlich charakterisiert ist, insoweit das er nicht mit dem normalen Zellmetabolismus besagter Patienten wechselwirkt, oder nur in einer nicht zellvergiftenden Weise, das er im Stande ist von Patienten in Dosierungen im Bereich von 600 mg/Tag bis 40 mg/Tag langfristig toleriert zu werden und worin der primäre Wirkstoff ohne weiteres vom Nierengewebe absorbiert wird und im Urin ohne nierenschädigende Seiteneffeke ausgeschieden wird.

7. Eine pharmazeutische Verbindung für orale Einnahme zur Verwendung in der Behandlung der Symptome erblicher Neuropathien des Bewegungsund Wahrnehmungssystems, der Riesen-Axonpathie, der diabetische Polyneuropathie oder dazu verwandter metabolische Symptomatik, präseniler Alzheimer Demenz, seniler Alzheimer Demenz, Down's Syndrom, Pick's Krankheit, Parkinson Krankheit, amyotrophischer lateraler Sklerose, Beschwerden die klinisch mit den obenerwähnten neurologischen Krankheiten verbunden sind, Huntington's Chorea, Ohrensausen, spinale Muskelatrophie, Friedreich Ataxie, Alkohol-bedingte Polyneuropathie, Multiple Sklerose, Ceroidlipofuscinose, altersbedingte Atrophie des periphären Nervensystems des Wahrnehmungs- und Bewegungstrakts, alterbedingte Atrophie des autonomen Nervensystems, einschließlich der Symptome der Hypoperistaltik des Verdauungstrakts, Zwerchfellhernie (partieller Nahrungsrückstau), Harninkontinenz, Atmungsbeschwerden aufgrund von Zwerchfellschwäche und verminderte autonome Sexualfunktionen, altersbedingte Atrophie der Nervenzellen des zentralen Nervensystems, Muskeldistrophie, altersbedingte pathophysiologisch bedingte Veränderungen des Kreislaufs, der Nieren und der Augenlinse, Arteriosklerose; die eine effective Menge wenigstens eines der primären Wirkstoffe aus dem Anspruch Nr. 1 enhält in Verbindung mit einem erforderlichem Zusatzmittel welches von der Gruppe der Antioxidans, Vitamine, einem Hormon, der chemisch zugeordneten Substanz welche die Drogenausscheidung von den Nieren fördert, dem Metabolit der Gefahr der Depletion ausgesetzt ist, und sulfohydrylhaltiger Substanzen aus dem Anspruch Nr. 1, wahlweise in Verbindung mit einem pharmazeutisch akzeptablem Träger dafür.

## Revendications

1. Utilisation d'un agent primaire; ledit agent primaire correspondant à la forme acide libre, au sel pharmaceutiquement acceptable, à un isomère à cycle benzénique, au dérivé ester d'acide carboxylique, ou au dérivé ester d'acide sulfonique d'une substance hydrophile, de masse molaire faible (comprise entre 100 à 1100) contenant un groupement fonctionnel de type amine primaire **caractérisée en ce qu'**elle est choisie parmi :
a. l'acide p-aminobenzoïque (APAB);
b. l'acide p-aminomethylbenzoïque et les dérivés analogues de formule H₂ N-(CH₂)ₙ -C₆ H₄ -COOH avec n = 2-30, y compris les isomères dont le groupement aminoalkyl est en position m- et o- du cycle benzénique et les isomères dont l'aminé n'est pas en position ω du groupement aminoalkyl ;
c. l'acide 4-amino-3-méthylbenzoïque et les autres dérivés de l'APAB ou leurs isomères benzéniques **caractérisés en ce que** de tels dérivés incluent : de 1 à 4 substitutions additionnelles du cycle aromatique par des groupes d'atomes choisis parmi les groupes méthyle, éthyle, ou autres chaînes hydrocarbonées comprenant jusqu'à 5 atomes de carbone ; un groupement hydroxyle substitué de structure -OCH₃, -OC₂H₅ ou autres éthers de masse molaire plus importante comprenant jusqu'à 5 atomes de carbone ;
d. l'acide 4-amidinobenzoïque, H₂N-C(=NH)C₆H₄-COOH, et ses dérivés de formule : 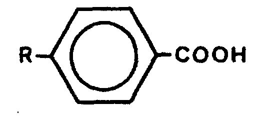 où R= -NHC(=NH)NH₂, CH₂ NHC(=NH)NH₂, ou (CH₂)ₙ NHC(=NH)NH₂ avec n = 2-10 ;
e. l'acide p-aminophénylacétique et les dérivés analogues de formule H₂ N-(CH₂)ₙ-C₆ H₄ -CH₂ -COOH avec n = 1-30, ainsi que les isomères du groupement aminoalkyl possédant une chaîne latérale hydrocarbonée du type méthyle ou autre, et/ou les dérivés dont la chaîne latérale du groupement aminoalkyl est hydroxylée, et/ou les dérivés portant des substitutions par des groupes hydrocarbonés ou hydroxyles au niveau du carbone α du groupement acétate ;
f. l'acide 4-amidinophénylacétique, H₂ N-C(=NH)C₆ H₄ -CH₂ - COOH ;
g. l'acide 3,5 diaminobenzoïque et les autres isomères benzéniques diaminés;
h. l'acide 3,5 diaminoalkylbenzoïque et les autres isomères benzéniques, **caractérisés en ce que** le groupe aminoalkyl est du type H₂ N-(CH₂)ₙ- avec n = 1-30, ou H₂ N-(CH₂)ₘ-CHOH-(CH₂)ₙ- avec m et n = 0-15, leurs isomères des chaînes hydrocarbonées étant inclus ;
i. l'acide p-aminosalicylique, et les dérivés amine et hydroxyle isomèriques, ainsi que les dérivés **caractérisés en ce que** l'hydroxyl a été substitué par un groupement méthoxy ou alcoxy ayant de 2 à 10 atomes de carbone ;
j. l'acide 4-amino-2-sulfobenzoïque et ses dérivés, y compris les isomères benzéniques et les dérivés dont le groupement amino a été remplacé par un groupement aminoalkyl ayant de 1 à 10 atomes de carbone, et les dérivés dont le groupement carboxylique acide est remplacé par un groupe -(CH₂ )ₙ -COOH avec n = 1-10 ; en vue de préparer une composition pharmaceutique destinée à l'administration orale en vue de traiter les symptômes des neuropathologies motrices et sensorielles héréditaires ; neuropathie à axone géant ; polyneuropathie diabétique ou symptomatologies métaboliques reliées à celui-ci ; démence présénile d'Alzheimer ; démence sénile d'Alzheimer ; syndrome de Down (ou trisomie 21) ; maladie de Pick ; maladie de Parkinson ; sclérose latérale amyotrophique ; désordres cliniques liés aux pathologies neurologiques susmentionnées ; maladie de Huntington ; tinnitus ; amyotrophie spinale ; ataxie de Friedreich ; polyneuropathie alcoolique ; sclérose en plaques ; ceroide lipofuschinose ; atrophie des fibres nerveuses périphériques sensorielles et motrices liée à l'age : atrophie des fibres nerveuses du système autonome liée à l'age incluant les symptômes d'hypoperistalsis du tractus alimentaire ; hernie hiatale (régurgitation partielle des aliments) ; incontinence urinaire ; insuffisance respiratoire due à la faiblesse du diaphragme et diminution de la fonction sexuelle autonome ; atrophie des neurones du système nerveux central liée à l'age ; désordres de la dystrophie musculaire ; changements pathophysiologiques liés à la vieillesse survenant dans le système cardiovasculaire, les reins, et le cristallin ; ou l'athérosclérose ; en association avec au moins un co-agent requis **caractérisé en ce qu'**il est choisi parmi le groupe fermé des anti-oxydants correspondant à la vitamine E (α-tocophérol), sélénium, acide citrique, ubiquinol, acides aminés contenant du sélénium, glutathione et protéines ayant un groupe sulfhydryl, le groupe fermé des vitamines correspondant à la vitamine A, vitamine D, vitamine K et à la vitamine B-6, le groupe fermé des hormones correspondant à l'hormone de croissance humaine, le groupe fermé des agents de détoxication qui facilitent l'élimination rénale correspondant à la glycine, le groupe fermé des métabolites dont la carence présente un risque correspondant à l'acide pantothénique, le groupe fermé des substances contenant un groupe sulfhydryl correspondant à la cystéine, homocystéine, méthionine, et à l'acide thioctique (ou acide lipodomique) ; et accessoirement en association avec un co-agent **caractérisé en ce qu'**il est choisi parmi les agents excipients ou leurs équivalents fonctionnels.

2. Utilisation selon la revendication 1, **caractérisée en ce que** ledit pharmaceutique est sous la forme d'une unité de dosage servant à l'administration dudit agent primaire à des doses allant de 600 mg à 40 gr par jours.

3. Utilisation selon l'une quelconque des revendications 1 ou 2, **caractérisée en ce que** ledit agent primaire inhibe la réaction se produisant entre (1) les chaînes hydrocarbonés aromatiques ou aliphatiques contenant des fonctions carbonyles qui sont générées par la maladie ou sont reliées à celle-ci et (2) les structures intracellulaires qui, en absence dudit agent primaire, donnent naissance à des produits associés par des liaisons covalentes.

4. Utilisation selon la revendication 3, **caractérisée en ce que** les associations par liaison covalente prennent en addition au moins une structure neuropathologique **caractérisée en ce qu'**elle est choisie parmi :
a. les agrégats polymérisés de protéine structurale filamenteuses tel que l'accumulation de neurofilament ;
b. les agrégats hétérogènes de protéines tels que la dégénérescence neuro-fibrillaire ;
c. les agrégats de lipides et protéines amorphes tels que les plaques séniles ; ou
d. les granules de lipofuscine

5. Utilisation selon l'une quelconque des revendications 3 ou 4, **caractérisée en ce que** l'agent primaire est un composé hydrosoluble, de masse molaire faible, possédant au moins un groupement de type amine primaire destiné à réagir avec les groupements carbonyle en vue de générer des produits liés de façon covalente appelés bases de Schiff, et **caractérisée en ce que** l'agent primaire est sélectionné parmi les groupes définis dans la revendication 1.

6. Utilisation selon la revendication 3, **caractérisée en ce que** l'agent primaire est choisi parce qu'il n'interagit pas avec le métabolisme normal de la cellule chez lesdits sujets humains ou qu'il interagit avec celui-ci de manière non toxique, **caractérisée en ce que** l'agent primaire est toléré par lesdits sujets humains à des doses allant de 600 mg à 40 gr par jours pour des durées prolongées, et **caractérisée en ce que** l'agent primaire est aisément absorbé par le tissu rénal et sécrété dans l'urine sans conséquences néphrotoxiques.

7. Une composition pharmaceutique destinée à l'administration orale en vue de traiter les symptômes des neuropathologies motrices et sensorielles héréditaires ; neuropathie à axone géant ; polyneuropathie diabétique ou symptomatologies métaboliques reliées à celui-ci ; démence présénile d'Alzheimer ; démence sénile d'Alzheimer ; syndrome de Down (ou trisomie 21) ; maladie de Pick ; maladie de Parkinson ; sclérose latérale amyotrophique ; désordres cliniques liés aux pathologies neurologiques susmentionnées ; maladie de Huntington ; tinnitus ; amyotrophie spinale ; ataxie de Friedreich ; polyneuropathie alcoolique ; sclérose en plaques ; ceroide lipofuschinose ; atrophie des fibres nerveuses périphériques sensorielles et motrices liée à l'age ; atrophie des fibres nerveuses du système autonome liée à l'age incluant les symptômes d'hypoperistalsis du tractus alimentaire ; hernie hiatale (régurgitation partielle des aliments) ; incontinence urinaire ; insuffisance respiratoire due à la faiblesse dû diaphragme et diminution de la fonction sexuelle autonome ; atrophie des neurones du système nerveux central liée à l'age ; désordres de la dystrophie musculaire ; changements pathophysiologiques liés à la vieillesse survenant dans le système cardiovasculaire, les reins, et le cristallin ; ou de l'athérosclérose ; comprenant une quantité efficace d'au moins un agent primaire tel que défini dans la revendication 1 combiné avec le co-facteur requis **caractérisé en ce qu'**il est choisi parmi le groupe des anti-oxydants, vitamines, hormones, agents de détoxication qui facilitent l'élimination rénale des drogues, métabolites dont la carence présente un risque, et des substances contenant un groupe sulfhydryl tel que défini dans la revendication 1 occasionnellement combiné à un agent transporteur pharmaceutiquement acceptable.
